(19) Europäisches Patentamt
European Patent Office
Office européen des brevets

(11) Publication number: **0 156 193**
**B1**

# (12) EUROPEAN PATENT SPECIFICATION

(45) Date of publication of patent specification: **27.12.90**

(21) Application number: **85102399.4**

(22) Date of filing: **04.03.85**

(51) Int. Cl.[5]: **A 61 K 31/435,**
C 07 D 491/16, A 23 K 1/17,
C 12 P 17/18 // C12N1/20
,(C07D491/16, 319:00, 311:00,
221:00),(C12P17/18,
C12R1:03)

## (54) Compounds and compositions for treating protozoal infections with a novel antibiotic.

(30) Priority: **26.03.84 US 593160**
**26.03.84 US 593162**
**26.03.84 US 593152**
**26.03.84 US 593159**

(43) Date of publication of application:
**02.10.85 Bulletin 85/40**

(45) Publication of the grant of the patent:
**27.12.90 Bulletin 90/52**

(84) Designated Contracting States:
**AT BE CH FR GB IT LI LU NL SE**

(56) References cited:
**DE-A-2 621 690**
**US-A-4 277 478**
**US-A-4 407 946**

**The file contains technical information submitted after the application was filed and not included in this specification**

(73) Proprietor: **AMERICAN CYANAMID COMPANY**
**1937 West Main Street P.O. Box 60**
**Stamford Connecticut 06904-0060 (US)**

(72) Inventor: **Lee, Taikwang Michael**
**11 Rambling Brook Drive**
**Holmdel New Jersey 07733 (US)**
Inventor: **Borders, Donald Bruce**
**13 Heatherhill Lane**
**Suffern New York 10901 (US)**
Inventor: **Goodman, Joseph Jacob**
**134 Grotke Road**
**Spring Valley New York 10977 (US)**
Inventor: **Testa, Raymond Thomas**
**Rockledge Place**
**Cedar Grove New Jersey 07009 (US)**
Inventor: **Maiese, William Michael**
**891 Sherwood Road**
**Bridgewater New Jersey 08807 (US)**
Inventor: **Labeda, David Paul**
**2320 W. Pintura Court**
**Peoria Illinois 61614 (US)**
Inventor: **Kantor, Sidney**
**44 Martin van Buren Drive**
**Cranbury New Jersey (US)**
Inventor: **Kennett, Robert Lee, Jr.**
**Box 203, R.D.I.**
**Lambertville New Jersey (US)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 156 193 B1**

Courier Press, Leamington Spa, England.

(72) Inventor: Carter, Guy Thomas
8 Prairie Avenue
Suffern New York 10901 (US)

(74) Representative: Wächtershäuser, Günter, Dr.
Tal 29
D-8000 München 2 (DE)

**Description**

Background of the Invention

The present invention relates to compounds and compositions for preventing, treating or controlling protozoal infections in warm-blooded animals by administering thereto an effective amount of the antibiotic LL—D4207α or LL—D42067β.

Coccidiosis is one of the most important of the protozoan parasitic diseases which plague the meat-producing industry. It is responsible for significantly greater losses to the poultry industry than from any other protozoan disease and is likewise responsible for substantial economic loss among a wide variety of farm, companion and game animals.

This disease is caused by protozoan parasites which infet the host animals causing them to lose weight, reduce their feed efficiency; and, in may instances, die. In poultry, these protozoan parasites are generally of the genus *Eimeria*; six species of which have been shown to be primary causative agents for the disease in poultry. These six species are: *Eimeria tenella, Eimeria necatrix, Eimeria mivati, Eimeria maxima, Eimeria brunetti,* and *Eimeria acervulina.*

Although coccidiosis has been recognized, for many years, as one of the most important diseases confronting the meat-producing industry, nevertheless, heretofore no entirely satisfactory method of control of the disease has been provided.

Summary of the Invention

It is an object of this invention to provide novel compositions effective for the control of protozoan infections in meat-producing animals.

The present invention relates to novel compounds and compositions effective for controlling, treating, minimizing, preventing, ameliorating, or curing protozoal infections in farm, companion, and game animals, particularly in meat-producing animals such as poultry, cattle, sheep, swine, and rabbits, and companion animals such as rabbits, dogs, and cats.

The antiobiotics which are useful in the compounds and compositions of this invention are LL—D42067α and LL—D42067β, NRRL *15734.*

The antibiotic, designated LL—D42067α, NRRL *15734,* has the following structural formula:

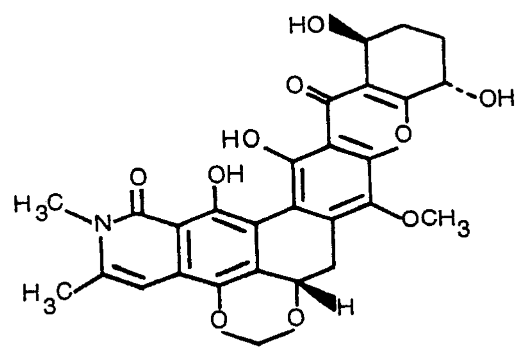

LL–D42067*α*

The antibiotic, designated LL—D42067β, NRRL *1534,* has the following structural formula:

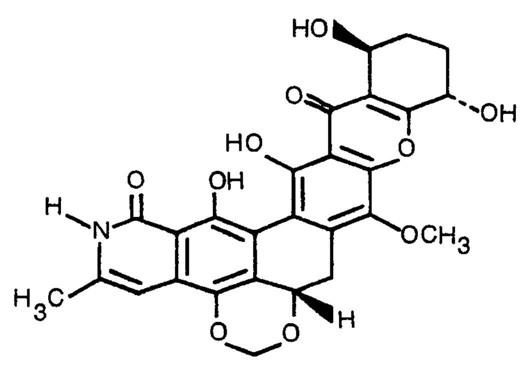

LL–D42067*β*

Description of Drawings

Figure I: Characteristic ultraviolet absorption spectra of compound designated LL—D42067α, NRRL *5734.*

Figure II: Characteristic infrared absorption spectrum of compound designated LL—D42067α, NRRL *15734.*

Figure III: Characteristic proton nuclear magnetic resonance spectrum of compound designated LL—D42067α, NRRL *15734,* in $CDCl_3$ solution.

Figure IV: Characteristic carbon-13 nuclear magnetic resonance spectrum of compound designated LL—D42067α, NRRL *15734,* in DMSO solution.

Figure V: Characteristic proton to carbon-13 chemical shift correlation of compound designated LL—D42067α, NRRL *15734,* DMSO solution.

Figure VI: Characteristic ultraviolet absorption spectra of compound designated LL—D42067β, NRRL *5734.*

Figure VII: Characteristic infrared absorption spectrum of compound designated LL—D42067β, NRRL *15734.*

Figure VIII: Characteristic proton nuclear magnetic resonance spectrum of compound designated LL—D42067β, NRRL *15734,* in $CDCl_3$ solution.

Figure IX: Characteristic carbon-13 nuclear magnetic resonance spectrum of compound designated LL—D42067β, NRRL *15734,* in $CD_3$ $OD/CDCl_3$ solution.

Detailed Description of the Invention

It has been discovered that the above-mentioned antibiotics are especially effective for controlling coccidiosis caused by *Eimeria* species in meat-producing animals, particularly in poultry such as chickens, turkeys, ducks, geese, quail, and pheasants, and in cattle, sheep, swine, and rabbits.

It is also anticipated that the antibiotic compositions of this invention will prove to be effective for controlling malaria, toxoplasmosis, and sarcosporidiosis in warm-blooded animals since the causative agents for such diseases are protozoan infections and are biologically related to *Eimeria.*

In practice, the present invention involves the method of preventing, controlling, or treating protozoal infections, such as coccidiosis, in warm-blooded animals by administering thereto, a prophylactically, pharmaceutically, or therapeutically effective amount of the antibiotic compound designated LL—D42067α or LL—D42067β, NRRL *15734,* or a pharmaceutically and pharmacologically acceptable salt thereof.

Although administration of the compound for coccidiosis will generally be most practical in or with the feed or in the drinking water, the above-said compound, or a pharmaceutically and pharmacologically acceptable salt thereof, may also be administered to individual hosts in the form of tablets, drenches, gels or capsules, or by injection in the form of a paste, gel, pellet, or solution. These latter methods of administration are, of course, less practical for the treatment of large groups of animals, but they are quite practical for use on a small scale or on an individual basis.

When the antibiotics, LL—D42067α or LL—D42067β, are used as prophylactic or therapeutic treatment for coccidiosis in poultry, generally about 0.1 ppm to 10.0 ppm, and preferably, 0.5 ppm to 3.0 ppm of the antibiotic LL—D42067α or LL—D42067β, administered in the diet or drinking water of the poultry, or inhibiting coccidiosis in said animals.

As previously indicated, the antibiotic LL—D42067α or LL—D42067β, or a pharmaceutically and pharmacologically acceptable salt thereof, may also be employed as a prophylactic, pharmaceutical, or therapeutic treatment for the control, prevention, or inhibition of protozoal infections in other warm-blooded animals such as cattle, sheep, and swine. Generally, about 1.0 ppm to 100 ppm, and preferably 5 ppm to 50 ppm, of the antibiotic is effective for controlling protozoal infections, such as coccidiosis, in these larger animals.

Medicated poultry feeds useful in the method of the present invention are usually prepared by thoroughly admixing 0.0001% by weight to 0.0005% by weight of the antiobiotic LL—D42067α or LL—D42067β with a nutritionally balanced poultry feed, as for example, the chick feed described in the examples hereinafter.

Medicated cattle, sheep, or swine feed can be prepared in the same manner described above for the poultry feed excepting that 0.0001% by weight to 0.01% by weight of the antibiotic is admixed with the cattle, sheep, or swine feed.

When using the compound of the invention for the prevention or control of coccidiosis, the active anticoccidial agent is generally first prepared as an animal feed premix. The premix usually contains a relatively high percentage of the anticoccidial agent and is generally blended with the animal's feed just prior to administration. If desired, the feed premix may also be applied as a top dressing for the animal's daily ration.

Feed premixes or concentrates, useful in the practice of the present invention, may be prepared by admixing about 0.1% to 5.0% by weight of the above-identified antibiotic, or a pharmaceutically and pharmacologically acceptable salt thereof, with about 99.9% to 95% by weight of a suitable carrier or diluent. Carriers suitable for use to make up the feed supplement compositions include the following: alfalfa meal, soybean meal, cottonseed oil meal, linseed oil meal, sodium chloride, calcium carbonate, calcium sulfate, cornmeal, cane molasses, urea, bone meal, corncob meal and rice hull meal. The carrier promotes an essentially uniform distribution of the active ingredient in the finished feed into which the supplement is blended. It thus performs an important function by ensuring proper distribution of the active ingredient, i.e., about 0.1 ppm to 100 ppm thereof, throughout the feed. This is equivalent to 0.00001% to

0.01% by weight of the active ingredient in the finished feed. In practice, usually one or pounds of premix is added per ton of feed to obtain the desired level of antibiotic in the finished feed.

If the supplement or premix is used as a top dressing for feed, it likewise helps to ensure uniformity of distribution of the active material across the top of the dressed feed.

Since the compounds of this invention and their pharmaceutically and pharmacologically acceptable salts are relatively insoluble in water, it is generally desirable, when administering the compounds in the animal's drinking waster, to dissolve the active compounds in an organic solvent such as methanol, ethanol, acetone, DMSO, oleic acid, linoleic acid or propylene glycol, and admix with the solution a small amount of surfactant and/or dispersing agent to assure solution and/or dispersion of the active ingredient in the animal's drinking water.

Advantageously, where the treatment of a small number of the larger meat-producing animals is required to control a protozoan infection therein, the antibiotic LL—D42067α or LL—D42067β or a pharmaceutically or pharmacologically acceptable salt thereof may be orally administered, on a daily basis, to the host animal in the form of a medicated gel.

The medicated gel may be prepared by admixing a medicated gellant phase with an aqueous buffer phase under reduced pressure 33.25—665 mbar (25—50 mm Hg) at ambient temperature 20°C—60°C. The gellant is prepared by dissolving or dispersing 0.004% to 4.5% by weight based on the final formulation of the antibiotic LL—D42067α or LL—D42067β or a pharmaceutically or pharmacologically acceptable salt thereof, in 14% to 31% by weight of propylene glycol based on the final formulation and about 15% to 50% by weight of the gellant at 60°C to 80°C. Alternatively, the gellant phase may be prepared completely at ambient temperature as hereinbefore described.

The gellant phase may be prepared by slurrying 0.004% by weight to about 4.5% by weight of the antibiotic LL—D42067α or LL—D42067β or a pharmaceutically or pharmacologically acceptable salt thereof, with the gellant 15% to 50%, and preferably 15% to 35% by weight of formulation in propylene glycol 14% to 30% by weight for 15 minutes to one hour under reduced pressure 33.25—66.5 mbar (25—50 mm Hg) at room temperature. The gellant selected is a nonionic surfactant of structure α-hydro-Ω-hydroxy-poly(oxyethylene)poly(oxypropylene)poly(oxyethylene) block copolymer, average molecular weight 12,500; mp 56°C; Brookfield viscosity of 3,100 at 77°C; surface tension of 0.1% aqueous solution: 40.6 dynes/cm (measured with a du Nouy tensiometer).

An aqueous buffer solution may then be prepared for dissolving 1.5% by weight of citric acid and 1.0% by weight of trisodium citrate in about 3% by weight to about 25% by weight and preferably 6% to 12% by weight of final formulation in deionized or distilled water used in amounts of from 15% by weight to 50% by weight and preferably 35% to 45% by weight of formulation. This buffered solution provides a pH range at which long term chemical stability of the components of the gel formulation is achieved, i.e., pH 3—3.5.

Optional components which may be incorporated into the above solution at this stage are:

a. Benzyl alcohol added in amounts of from about 0.5% by weight to about 1.5% by weight and preferably 1.5% by weight of formulation as an antimicrobial preservative;

b. the yellow dye C.I. Acid yellow No. 23; ("tartrazine;" F. D. and C yellow No. 5; 4,5-dihydro-5-oxo-1-(4-sulfophenyl)-4-[(sulfophenyl)azo]-1H-pyrazole-3-carboxylic acid trisodium salt) used as coloring agent in amounts of from 0.01% by weight to 0.03% by weight and preferably 0.01% by weight of formulation;

c. an antifoaming agent comprising a mixture of dimethylpolysiloxanes of structure:

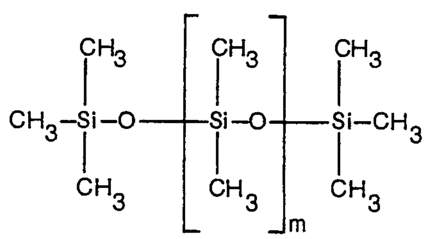

and silica gel, wherein the calculated average value of m is 200—350, the mixture is a water-white viscous oil-like liquid; d=0.965—0.970; $n_D^{25}$ about 1.404; viscosity about 60,000 centistokes used in amounts of from 0.001—0.02% by weight and preferably 0.02% by weight of formulation.

The medicated gel is prepared by simply mixing either of the above-gellant phases and the aqueous solution from one-half to two hours under reduced pressure from 13.3—133.0 mbar (10—100 mm Hg) and preferably 33.25—66.5 mbar (25—50 mm Hg), at ambient temperatures of from 20°C to 60°C without the requirements of either additional heating or cooling. This procedure gives an air-free gel which is suitable for administering exact dosages of anticoccidial by volume. When careful control of dosage active ingredients to be administered by volume is not necessary, and when the presence of air in the gel is acceptable in the final formulation, the preparation may be carried out at pressures up to and including atmospheric pressure.

By the method, a typical gel of the invention may be prepared by dissolving 4.5 g of the antibiotic LL—D42067α (or 4.5 g of the antibiotic LL—D42067β), 1.5 g citric acid monohydrate, 1.0 g sodium citrate

dihydrate, 1.5 g of benzyl alcohol, and 0.01 g of the yellow dye C.I. Acid yellow No. 23 in 42 g of water. Next, a solution of the above gellant 28 g in propylene glycol 21.99 g is prepared by mixing at 60°C. Then the solutions are mixed togetheer under 33.25—66.5 mbar (25—50 mm Hg) until a homogeneous mixture is obtained at 20°C to 60°C without additional heating or cooling. The gel formed has a gelation temperature range of from −15°C to −18°C; viscosity of the gel is 0.51 × $10+^{6}$; and the water gellant ratio is 1.5/1.0.

When 6.3 grams of this medicated gel are orally administered to 90.8 kg (200 pound) fattening steer on a daily basis, said steer receives approximately 3 mg/kg of body weight/day of the protozoacidically-effective antibiotic LL—D42067α or antibiotic LL—D42067β.

In practice, generally 0.03 mg/kg/day to 3.0 mg/kg/day is effective for controlling protozoan infections in cattle, sheep and swine. For smaller companion animals, rates as low as 0.003 mg/kg of body weight/day may be employed.

The structure of LL—D42067α has been elucidated by x-ray crystallography, and the relative stereochemistry of this compound is shown above.

The structure of LL—D42067β has been elucidated by x-ray crystallography, and the relative stereochemistry of this compound is shown above.

The physico-chemical characteristics of LL—D42067α are described below:

1) Molecular weight: 535 (FAB—MS);
2) Molecular formula: $C_{28}H_{25}NO_{10}$;
3) Specific optical rotation $[\alpha]_D^{26}$ = +836±40° (C 0.3, DMF);
4) Ultraviolet absorption spectra: as shown in Figure I

$UV_{MAX}^{CH_3OH}$ = 215nm (ε 13,200)
254nm (ε 15,000)
320nm (ε 5,100)
395nm (ε 11,400)

$UV_{MAX}^{0.1N\ HCl}$ = 213nm (ε 27,100)
253nm (ε 34,500)
321nm (ε 12,200)
374nm (ε 21,100)
389nm (ε 22,900)

$UV_{MAX}^{0.1N\ NaOH}$ = 217nm (ε 42,100)
253nm (ε 13,900)
312nm (ε 5,700)
395nm (ε 10,700)

5) Infrared absorption spectrum: as shown in Figure II (KBr disc): 1650, 1598, 1543, 1470, 1440, 1260, 1195, 1020 $cm^{-1}$;

6) Proton nuclear magnetic resonance spectrum ($CDCl_3$): as shown in Figure III, and described in Table I;

7) Carbon-13 nuclear magnetic resonance spectrum (DMSO); as shown in Figure IV and described in Table II and

8) Proton to carbon-13 chemical shift correlation map (DMSO): as shown in Figure V.

The physico-chemical characteristics of LL—D42067β are described below:

1) Molecular weight: 521 (FAB—MS);
2) Molecular formula: $C_{27}H_{23}NO_{10}$;
3) Specific optical rotation: $[\alpha]_D^{26}$ = +770 ± 10° (C 0.165, DMF);
4) Ultraviolet absorption spectra: as shown in Figure I

$UV_{MAX}^{CH_3OH}$ = 212nm (ε 28,200)
253nm (ε 33,200)
318nm (ε 13,200)
378nm (ε 19,700)
393nm (ε 21,700)

$UV_{MAX}^{0.1N\ HCl}$ = 211nm (ε 13,900)
253nm (ε 18,200)
318nm (ε 7,140)
372nm (ε 9,950)
388nm (ε 10,500)

$UV_{MAX}^{0.1N\ NaOH}$ = 216nm (ε 31,200)
251nm (ε 11,700)

5) Infrared absorption spectrum: as shown in Figure VII (KBr disco): 3400, 1646, 1620, 1545, 1463, 1252, 1195, 1020 $cm^{-1}$;

6) Proton nuclear magnetic resonance spectrum ($CDCl_3$) as shown in Figure VIII and described in Table III;

7) Carbon-13 nuclear magnetic resonance spectrum ($CD_3OD/CDCl_3$) as shown in Figure IX and described in Table IV.

TABLE I

Proton NMR Data for LL—D42067α

| δ* | No. of Hydrogen** | Multiplicity*** | J (H) |
|---|---|---|---|
| 1.88 | 2 | m | |
| 2.34 | 2 | m | |
| 2.45 | 3 | s | |
| 2.58 | 1 | m | |
| 3.62 | 3 | s | |
| 3.72 | 1 | d,d | 4.64, 14.22 |
| 3.88 | 3 | s | |
| 4.80 | 2 | m | |
| 5.08 | 1 | m | |
| 5.32 | 1 | d | 5.81 |
| 5.55 | 1 | d | 5.81 |
| 6.70 | 1 | s | |
| 12.76 | 1 | s | |
| 13.58 | 1 | s | |

*$CDCl_3$, ppm downfield from TMS
**Spectrum in DMSS-$d_6$, shows two additional absorptions at 4.55 (s) and 5.91 (d) ppm
***s = singlet; d = doublet; t = triplet; m = multiplet

TABLE II

Carbon-13 NMR Data for LL—D42067α

| Carbon | Chemical Shift (ppm)* | Carbon Type |
|---|---|---|
| 1 | 20.4 | $CH_3$ |
| 2 | 25.4 | $CH_2$ |
| 3 | 25.8 | $CH_2$ |
| 4 | 29.0 | $CH_2$ |
| 5 | 30.4 | $CH_3$ |
| 6 | 58.5 | CH |
| 7 | 61.6 | $CH_3$ |
| 8 | 63.3 | CH |
| 9 | 71.7 | CH |
| 10 | 90.4 | $CH_2$ |
| 11 | 100.0 | CH |
| 12 | 109.2 | q** |
| 13 | 109.7 | q |
| 14 | 111.0 | q |
| 15 | 113.7 | q |
| 16 | 119.0 | q |
| 17 | 125.8 | q |
| 18 | 126.6 | q |
| 19 | 134.8 | q |
| 20 | 135.3 | q |
| 21 | 136.1 | q |
| 22 | 141.3 | q |
| 23 | 147.9 | q |
| 24 | 151.1 | q |
| 25 | 152.5 | q |
| 26 | 165.4 | q |
| 27 | 165.6 | q |
| 28 | 182.5 | q |

*DMSO-$d_6$, ppm downfield from TMS
**q = quarternary

TABLE III

Proton NMR Data for LL—D42067β

| δ* | No. of Hydrogen | Multiplicity** | J (H) |
|---|---|---|---|
| 1.88 | 2 | m | |
| 2.30 | 2 | m | |
| 2.38 | 3 | s | |
| 2.57 | 2 | m | |
| 3.69 | 1 | d,d | 4.5, 14.3 |
| 3.87 | 3 | s | |
| 4.79 | 1 | m | |
| 4.81 | 1 | d,d | 4.5, 12.7 |
| 5.06 | 1 | m | |
| 5.32 | 1 | d | 5.8 |
| 5.53 | 1 | d | 5.8 |
| 6.54 | 1 | s | |
| 10.1 | 1 | s | |
| 12.8 | 1 | s | |
| 12.9 | 1 | s | |

*$CDCl_3$, ppm downfield from TMS
**s = singlet; d = doublet; t = triplet; m = multiplet

TABLE IV

Carbon-13 NMR Data for LL—D42067β

| Carbon | Chemical Shift (ppm)* |
|---|---|
| 2 | 25.9 |
| 3 | 26.9 |
| 4 | 29.7 |
| 5 | 62.0 |
| 6 | 62.2 |
| 7 | 65.10 |
| 8 | 72.8 |
| 9 | 91.1 |
| 10 | 100.1 |
| 11 | 110.5 |
| 12 | 110.6 |
| 13 | 111.7 |
| 14 | 114.7 |
| 15 | 119.2 |
| 16 | 127.2 |
| 17 | 128.8 |
| 18 | 136.1 |
| 19 | 136.3 |
| 20 | 137.0 |
| 21 | 138.1 |
| 22 | 148.9 |
| 23 | 151.9 |
| 24 | 153.0 |
| 25 | 165.3 |
| 26 | 167.2 |
| 27 | 183.6 |

*$CD_3OD/CDCl_3$, ppm downfield from TMS

The protozoacidially-effective compounds of this invention, designated LL—D42067α and LL—D42067β, are formed during the cultivation under controlled conditions of a new strain of a new subspecies of *Actinomadura madurae.* The new strains are maintained in a culture collection of the Medical Research Division, American Cyanamid Company, Pearl River, New York as culture number LL—D42067α and LL—D42067β. Viable cultures of these new microorganisms have been deposited with the Patent Culture Collection Laboratory, Northern Regional Research Center, U.S. Department of Agriculture, Peoria, Illinois 61604, and have been added to its permanent collection. They are freely available to the public in this depository under its accession number NRRL 15734.

Cultures LL—D42067α and LL—D42067β were isolated from a soil sample from San Simao, Brazil. The cultures were taxonomically characterized and were identified as new subspecies of *Actinomadura madurae,* designated *Actinomadura madurae* subspecies *simaoensis.*

Observations were made of the cultural, physiological and morphological features of the culture in accordance with the methods detailed by Shirling and Gottlieb [Intern. J. System. Bacteriol., *16*:313—340 (1966)], and Gordon, *et al.* [Intern. J. System. Bacteriol., *24*:54—63 (1974)]. The chemical composition of the cell walls of the cultures were determined using the method of Lechevalier, *et al.* [Adv. Appl. Microbiol., *14*:47—72 (1971)]. Details were recorded in Tables V—VII, and a general description of the cultures are given below. Underscored descriptive colors are taken from Kelly and Judd [Nat. Bur. Stand., Spec. Publ., 440 (1976)] and the accompanying Intersociety Color Council, National Bureau of Standards Centroid Color Charts.

*Growth Characteristics*

Table V describes the cultural characteristics of cultures LL—D42067 on various agar media which were selected from those recommended by the International Streptomyces Project Committee (hereinafter referred to as "ISP").

*Micromorphology*

Microscopic examination of the strain showed it to form short chains of conidia on aerial hyphae which were slightly hooked to short-spirals (up to three turns). The spore surfaces were smooth when observed by electron microscopy, distinguishing this isolate form *A. verrucosopora.*

*Cell Wall Composition*

Whole cell analyses showed the strain to contain *meso* diaminopimelic acid (DAP) and the sugar 3-*O*-methyl-*D*-galactose (madurose); thus it falls into whole cell pattern type B. The cell wall composition was of the type III (*meso* DAP, glutamic acid, alanine, muramic acid and glucosamine) and the phospholipid pattern of type PIV (phosphatidyl ethanolamine and/or methylethanolamine plus unknown glucosamine-containing phospholipids). These data support the assignment of the strain to the genus *Actinomadura.* The PIV phospholipid type is not typical for *A. madurae,* which is usually PI.

*Physiological Reactions*

The physiological reactions of strains LL—D42067 and LL—D42067 were examined using both the ISP system, Shirling and Gottlieb [Inter. J. Syst. Bacteriol., *16*:313—340 (1966)] and the Gordon tests, Gordon, *et al.* [Intern. J. Syst. Bacteriol., *24*:54—63 (1974)]. The utilization pattern of the strain on ISP carbohydrate media is given in Table VI, along with those of other members of the genus reacting similarly. Culture LL—D42067 resembles the *Actinomadura madurae* and *Actinomadura verrucosopora* groups. As indicated above, however, it differs from *Actinomadura verrucosopora* in having smooth spore walls. A comparison of reactions in the Gordon test series of *Actinomadura madurae* (Gordon's data; see reference above) and LL—D42067, summarized in Table VII, revealed differences only in the amylase production and acid from glycerol and raffinose. Since amylase production and raffinose utilization have been found to be variable in *Actinomadura madurae* [Goodfellow, N., *et al.,* J. Gen. Microbiol., *112*:95—111 (1979)], the glycerol reaction remains the only physiological difference of LL—D42067 from this taxon.

Since strain LL—D42067 is the same as *Actinomadura madurae* in all properties evaluated except for its glycerol reactions and its PIV phospholipid pattern, it has been assigned to the taxon *Actinomadura madurae* as a subspecies designated *Actinomadura madurae* subspecies *simaoensis.*

TABLE V

Cultural Characteristics of LL—D42067 *Actinomadura madurae* supspecies *simaoensis* on ISP Morphological Media

| Agar Medium | Aerial Mycelium | Vegetative Mycelium | Soluble Pigment |
|---|---|---|---|
| Yeast extract, Malt extract (ISP 2) | White sparse | *Medium orange-brown*—I53* | None |
| Inorganic Salts Starch (ISP 4) | Colorless | Colorless | None |
| Glucose Asparagine (ISP 5) | Colorless | Colorless | None |
| Oatmeal (ISP 3) | Sparse pinkish-white | *Light orange-brown*—I52* | None |

I = ISCC Color charts

TABLE VI

Comparison of Carbohydrate Utilization Reactions of LL—D42067 with Related *Actinomadura* spp.

| Carbohydrate | LL—D42067 | *A. madurae* (a) | *A. verrucosospora* (a) (b) |
|---|---|---|---|
| *L*-arabinose | + | + | + |
| *D*-fructose | + | + | + |
| I-inositol | – | variable | variable |
| *D*-mannitol | + | + | + |
| raffinose | – | – | – |
| rahmnose | + | + | + |
| sucrose | + | + | + |
| *D*-xylose | + | + | + |

(a) Goodfellow, M., *et al.,* J. Gen. Microbiol., *112*:95—111 (1979).
(b) Nonomura, H. and O'Hara, Y., J. Ferm. Technol., *49*:904—912 (1971).

TABLE VII

Gordon Test Reactions of LL—D42067

| | LL—D42076 | *A. madurae* (Gordon Data*) |
|---|---|---|
| *Degradation/Transformation of* | | |
| Casein | + | +(98) |
| Xanthine | − | − |
| Hypoxanthine | + | +(98) |
| Tyrosine | + | +(91) |
| Adenine | − | − |
| *Production of* | | |
| Amylase | − | + |
| Gelatinase | + | + |
| Phosphatase | − | ND |
| Nitrate Reductase | + | +(98) |
| Urease | − | − |
| Esculinase | + | +(98) |
| *Growth on/in* | | |
| 5% Sodium Chloride | − | ND |
| Salicylate | − | ND |
| Lysozyme Broth | − | −(91) |
| *Utilization* | | |
| Acetate | + | + |
| Benzoate | − | −(94) |
| Citrate | − | +(83) |
| Lactate | + | ND |
| Malate | + | +(84) |
| Mucate | − | − |
| Oxalate | − | ND |
| Propionate | − | ND |
| Pyruvate | + | ND |
| Succinate | + | +(83) |
| Tartrate | − | − |

TABLE VII (continued)

| | LL—D42076 | *A. madurae* (Gordon Data*) |
|---|---|---|
| *Growth at* | | |
| 10°C | – | – |
| 45°C | + | –(66) |
| 53°C | – | – |
| *Acid from* | | |
| Adonitol | + | +(91) |
| Arabinose | + | + |
| Cellobiose | + | + |
| Dextrin | + | ND |
| Dulcitol | – | – |
| Erythritol | – | – |
| Fructose | + | ND |
| Galactose | + | +(84) |
| Glucose | + | + |
| Glycerol | – | + |
| Inositol | – | +(60) |
| Lactose | – | +(55) |
| Maltose | – | +(53) |
| Mannitol | + | + |
| Mannose | + | +(94) |
| Melibiose | – | – |
| α-Methyl-*D*-glucoside | – | – |
| Raffinose | variable | – |
| Rhamnose | + | + |
| Salicin | + | ND |
| Sorbitol | – | – |
| Sucrose | + | ND |
| Trehalose | + | +(96) |
| Xylose | + | + |
| β-Methyl-*D*-xyloside | + | ND |

*Percentages of cultures showing reaction given in parentheses if not 100%.
ND = Not determined.

For the production of this new antibacterial and antiparasitic agent the present invention is not limited to this particular organism or to organisms fully answering the above growth and microscopic characteristics, which are given for illustrative purposes only. In fact, it is desired and intended to include the use of naturally-occurring mutants of this organism as well as induced mutants produced from this organism by various mutagenic means known to those skilled in the art such as exposure to nitrogen mustard, X-ray radiation, ultraviolet radiation, *N'*-methyl-*N'*-nitro-*N*-nitrosoguanidine and actinophages. It is also desired and intended to include inter- and intraspecific genetic recombinants produced by genetic techniques known to those skilled in the art such as, for example, conjugation, transduction and genetic engineering techniques.

The *in vitro* antimicrobial spectrum of LL—D42067β was determined by the agar plate dilution method with Mueller-Hinton agar and an inoculum of each test organism of approximately $10^4$ colony forming units delivered by the Steers replicating device. The minimal inhibitory concentration (MIC in μg/ml was defined as the lowest concentration of LL—D42067β that inhibited visible growth after 18 hours incubation at 35°C.

The results, summarized in Table VIII show that LL—D42076β was active versus gram-positive bacteria and moderately active against yeasts.

TABLE VIII

Antimicrobial Spectrum of LL—D42067β

| Test Organism | | MIC (μg/ml) |
|---|---|---|
| *Candida albicans* | CA 300 | 512 |
| *Saccharomyces cerevisiae* | Y 15 | 512 |
| *Mycobacterium smegmatis* | ATCC 607 | 512 |
| *Bacillus subtilis* | ATCC 6633 | 4 |
| *Bacillus cereus* | LL No. 4 | ≤0.06 |
| *Enterococcus* | OSU 75—1 | 1 |
| *Enterococcus* | SM 77—15 | 2 |
| *Streptococcus faecalis* | ATCC 29212 | 1 |
| *Streptococcus mutans* | ATCC 27352—1 | 0.25 |
| *Streptococcus mutans* | BHI (b) | 0.25 |
| *Streptococcus sanquis* | G9B (a) | 0.5 |
| *Staphylococcus epidermidis* | CMC 83—56 | 0.5 |
| *Staphylococcus epidermidis* | ATCC 12228 | 0.25 |
| *Staphylococcus aureus* | Smith | 0.5 |
| *Staphylococcus aureus* | LL No. 14 | 0.5 |
| *Staphylococcus aureus* | LL No. 27 | ≤0.06 |
| *Staphylococcus aureus* | LL No. 45 | 0.12 |
| *Staphylococcus aureus* | ATCC 25923 | 0.25 |
| *Micrococcus luteus* | PC 1001 | ≤0.06 |
| *Escherichia coli* | No. 311 | 512 |
| *Escherichia coli* | ATCC 25922 | 512 |
| *Acinetobacter calcoaceticus* | STFD 79—17 | 512 |

The antibiotic LL—D42067β derives utility from its antibacterial and antiparasitic activities. For example, the antibiotic may be used in the suppression of intestinal bacterial flora, as a topical antibacterial agent or antiseptic against gram-positive bacteria and as a general disinfectant for surfaces such as instruments. It may also be useful as an antiprotozoal agent in the treatment of malaria. In addition to its antimicrobial and antiparasitic activity LL—D42067β is effective as an anticoccidial agent in poultry.

In therapeutic use, the compound of this invention may be administered in the form of conventional pharmaceutical composition appropriate for the intended use. Such compositions may be formulated so as to be suitable for oral or topical administration. The active ingredient may be combined in admixture with a nontoxic pharmaceutically acceptable carrier, which carrier may take a wide variety of forms depending on the form of the preparation desired for administration, *i.e.,* oral or topical.

*General Fermentation Conditions*

Cultivation of *Actinomadura madurae* subspecies *simaoensis* NRRL *15 73 4* may be carried out in a wide variety of liquid culture media. Media which are useful for the production of this novel antibiotic LL—D42067α include an assimilable source of carbon, such as dextrin, sucrose, molasses and glycerol; an assimilable source of nitrogen such as protein, protein hydrolysate, polypeptides, amino acids and corn steep liquor; and inorganic anions and cations, such as potassium, sodium, ammonium, calcium, sulfate, carbonate, phosphate and chloride. Trace elements such as boron, molybdenum and copper, are supplied as impurities of other constituents of the media. Aeration in tanks and bottles is supplied by forcing sterile air through or onto the surface of the fermenting medium. Further agitation in tanks is provided by a mechanical impeller. An antifoam agent such as silicone oil may be added as needed.

*General Procedure for the Isolation of LL—D42067α and LL—D42067β*

The LL—D42067α antibiotic is recovered from the fermentation broth by filtration through diatomaceous earth, extracted into a solvent such as methylene chloride and purified by column chromatography on silica gel, using the system hexane:ethyl acetate (80:20) to remove unwanted fats and then methylene chloride:1% acetic acid in methanol (9:1) to give a crude product.

This crude LL—D42067α is then purified by high performance liquid chromatography on a reverse phase column using the system acetonitrile:water:acetic acid (600:400:0.28).

Antibiotic LL—D42067β is recovered from the whole harvest mash by filtration through a medium such as diatomaceous earth, extraction into a solvent such as ethyl acetate, concentration to a syrup, partitioning between heptane and methanol and concentration of the methanol phase to a residue. This residue is triturated with hexane, then concentrated to a residue which is dissolved in a mixture of equal parts acetonitrile and water and then evaporated giving a precipitate. This precipitate is purified by preparative reverse phase high performance liquid chromatography (HPLC) using the system acetonitrile:water:acetic acid (3000:6000:5). The active fractions are combined, evaporated to an aqueous suspension and extracted with ethyl acetate which is evaporated to obtain the pure LL—D42067β.

Example 1

Inoculum Preparation

A typical medium used to grow the primary inoculum was prepared according to the following formula:

| | |
|---|---|
| Glucose | 10% |
| Dextrin | 2.0% |
| Yeast extract | 0.5% |
| N—Z Amine *A*®[1] | 0.5% |
| Calcium carbonate | 0.1% |
| Water qs | 100% |

[[1]A pancreatic digest of casein, registered trademark of Sheffield Chemical, Norwich, New York]

This medium was adjusted to pH 7.2 and then sterilized. A 100 ml portion of this sterile medium, in a 500 ml flask, was inoculated with mycelial scrapings from an agar slant of *Actinomadura madurae* subspecies *simaoensis* NRRL 15734. The medium was then placed on a rotary shaker and agitated vigorously at 210 rpm for 48—72 hours at 28°C. This primary inoculum was then used to inoculate 12 liters of the same sterile medium which was then grown at 28°C for 48 hours providing secondary inoculum.

Example 2
Fermentation

A fermentation medium of the following formulation was prepared:

| | |
|---|---|
| Sucrose | 3.0% |
| Soy flour | 1.5% |
| Corn steep liquor | 0.5% |
| Calcium carbonate | 0.5% |
| Water qs | 100% |

The medium was sterilized and inoculated at the rate of 12 liters of secondary inoculum from Example 1 per 300 liters of medium. The fermentation was conducted at 28°C with a sterile air flow of 200 liters per liter of mash per minute, agitation by an impeller operated at 230 rmp for 135—159 hours at which time the mash was harvested and filtered through diatomaceous earth.

Example 3
Isolation of LL—D42067α

The fermentation filtrate from three fermentations, conducted as described in Example 2, were combined, making a total of 1800 liters at pH 7.5, and extracted with 900 liters of methylene chloride. The organic phase was concentrated *in vacuo* to give 84.1 g of residue.

A 75.2 g portion of this residue was suspended in 300 ml of hexane:ethyl acetate (80:20) and allowed to seep into a glass column (5.08 cm × 50.8 cm); (2 inches × 20 inches) dry packed with silica gel. The column was eluted with a total of 4 liters of the same solvent mixture in order to remove fats and silicone oil and was then eluted with 4 liters of methylene chloride:1% acetic acid in methanol (9:1) collecting 15 ml fractions. The fractions were analyzed by thin-layer chromatography. Antibiotic LL—D42067α appeared visually as a yellow spot (Rf = 0.5) with the same solvent system. Fractions 31—60, which contained most of the antibiotic, were pooled and concentrated *in vacuo,* giving 11.1 g of a red residue.

A 5.5 g portion of the above residue was fractionated by high performance liquid chromatography [Prep LC System-500, Prep PAK—500/C18 cartridge, acetonitrile:water:acetic acid (600:400:0.28), 100 ml/minute, 5.5 g/30 ml/injection]. Thirty 200 ml fractions were collected. Analytical high performance liquid chromatographic analysis of the fractions showed the major portion of LL—D42067α was in fraction 5. Fraction 5 was allowed to stand overnight. The resulting yellow crystals were collected by decanting off the mother liquor (which was saved), washing the crystals with the mobile phase and air drying, giving 11 mg of LL—D42067α as yellow crystals.

The mother liquor was concentrated by slow evaporation. The resulting precipitate was collected by centrifugation giving 377 mg of LL—D42067α as a yellow amorphous solid.

The analytical HPLC conditions were:
Column: μ Bondapak® C18, 3.9 mm × 30 cm, Waters Associates
Mobile Phase: acetonitrile:water:acetic acid (400:600:0.28)
Detector: UV 254nm and UV 365nm, 0.2 AUFS
Flow Rate: 1.0 ml/minute
Retention Volume of LL—D42067α: 11.5 ml.

Example 4
Inoculum Preparation

A typical medium used to grow the various stages of inoculum was prepared according to the following formula:

| | |
|---|---|
| Dextrose | 1.0% |
| Dextrin | 2.0% |
| Yeast extract | 0.5% |
| NZ Amine *A*®' | 0.5% |
| Calcium carbonate | 0.1% |
| Water qs | 100% |

NZ Amine *A*®' (A pancreatic digest of casin; registered trademark of Sheffield Chemical, Norwich, New York).

This medium was sterilized. A 100 ml portion of this sterile medium, in a flask, was inoculated with mycelial scrapings from an agar slant of *Actinomadura madurae* subspecies *simaoensis* NRRL 15734. The medium was then agitated vigorously on a rotary shaker for 48—72 hours at 28°C providing primary inoculum. This primary inoculum was then used to inoculate 10 liters of the above sterile medium, which was then grown at 28°C for 48 hours, providing secondary inoculum. This secondary inoculum was then used to inoculate 250 liters of the above sterile medium, in a tank, which was grown for 48 hours at 28°C with a flow of sterile air of 200 liters per minute, providing tertiary inoculum.

Example 5

Fermentation

A fermentation medium of the following formulation was prepared.

| | |
|---|---|
| Sucrose | 3.0% |
| Soy flour | 1.5% |
| Corn sheep liquor | 0.5% |
| Calcium carbonate | 0.5% |
| Water qs | 100% |

This medium was sterilized and then inoculated with 125 liters of tertiary inoculum, prepared as described in Example 1, per 3000 liters of the above sterile fermentation medium. The fermentation was conducted at 28°C with a sterile air flow of 6.6 liters per liter of mash, agitation by an impeller operated at 110 rpm and the addition of silicone defoamer agent for 137 hours, at which time the mash was harvested.

Example 6

Isolation of LL—D42067β

A total of 4500 liters of harvest mash combined from two fermentations conducted essentially as described in Example 2 was combined with 1% of its volume of diatomaceous earth, mixed for one hour and then the pH was adjusted to 3.0±0.3 with concentrated hydrochloric acid. One half the mash volume of ethyl acetate was added and this mixture was stirred for 3 hours. Diatomaceous earth equal to 5% of the mash volume was added and the mixture was filtered. The ethyl acetate phase of the filtrate was separated, washed with 5% aqueous sodium bicarbonate and then concentrated to a syrup. This material was partitioned between heptane:methanol (2:1).

The 4.5 liters of methanol phase was concentrated to a residue which was triturated with hexane. The hexane was decanted and the residue concentrated to dryness. This material was purified by preparative reverse phase HPLC with the following conditions: (A 300 g silica-based octa decyl (C) bonded phase packing material (dimensions 5.7cm × 30cm), registered trademark of Wieley Associates, Inc., Milford, Mass).

| | |
|---|---|
| Column | A single PrepPak®-500/$C_{18}$ cartridge. |
| Mobile Phase 1: | Acetonitrile:water:acetic acid (8,000:12,000:10). |
| 2: | Acetonitrile:water:acetic acid (3,000:6,000:5). |
| Flow Rate: | 50 ml/minute. |
| Fractionation: | 200 ml/fraction. |
| Sample Load: | 2—3 g per 30 ml injection. |

Using mobile phase 1, LL—D42067β was found in fractions 7—10. These fractions were combined and evaporated *in vacuo* to remove the bulk of the acetonitrile. The resulting aqueous suspension was treated with an equal volume of ethyl acetate. The ethyl acetate phase was separated and sequentially washed with equal volumes of 5% aqueous sodium bicarbonate, 0.1N hydrochloric acid and twice with water. The organic phase was dried over anhydrous sodium sulfate and evaporated to a solid.

This solid was rechromatographed using mobile phase 2. In this mobile phase LL—D42067β was found in fractions 15—21 which were combined and treated as described above, giving purse LL—D42067β as a yellow solid.

Example 7

Evaluation of test compounds as anticoccidial agents

The usefulness of antibiotic LL—D42067α and LL—D42067β as an anticoccidial agent for chickens is demonstrated in the following tests.

The poultry diet employed in the test is as follows:

| | |
|---|---|
| Vitamin-amino acid premix | 0.5% |
| Trace minerals | 0.1% |
| Sodium chloride | 0.3% |
| Dicalcium phosphate | 1.2% |
| Ground limestone | 0.5% |
| Stabilized fat | 4.0% |
| Deyhdrated alfalfa, 17% protein | 2.0% |
| Corn gluten meal, 41% protein | 5.0% |
| Menhaden fish meal, 60% protein | 5.0% |
| Soybean oil meal, 44% protein | 30.0% |
| Ground yellow corn, fine to | 100.0% |

The vitamin-amino acid premix in the above feed composition is prepared from the following formulation. The expressions of quantity relate to units per kilogram of the finished feed composition.

| | |
|---|---|
| Butylated hydroxy toluene | 125.0 mg |
| dl-Methionine | 500.0 mg |
| Vitamin A | 3300.0 I.U. |
| Vitamin $D_3$ | 1100.0 I.C.U. |
| Ribiflavin | 4.4 mg |
| Vitamin E | 2.2 I.U. |
| Niacin | 27.5 mg |
| Panthothenic acid | 8.8 mg |
| Choline chloride | 500.0 mg |
| Folic acid | 1.43 mg |
| Menadione sodium bisulfate | 1.1 mg |
| Vitamin $B_{12}$ | 11.0 mcg |
| Ground yellow corn, fine to | 5.0 gm |

A mixed inoculum of 5000 sporulated oocysts of *Eimeria acervulina* and a sufficient number of occysts of *Eimeria tenella* to produce 60% to 75% mortality in untreated controls were given to one-day-old chicks, by direct inoculation into the crops of all chicks. The chicks were given free access to feed and water during the entire test period. Two days before inoculation, medicated feed with several levels of drug was

presented to the various groups of chicks. Seven days after inoculation, the tests were terminated and the chicks were weighed, necropsied, and their intestinal tracts examined for lesions. The results appear in the Tables below. These results show that improved survival of infected chicks is obtained when 0.2 ppm to 5.00 ppm of the antibiotic is administered to infected chicks in their diet. These levels also show a significant suppression of lesions due to *E. tenella* and *E. acervulina.*

TABLE IX
Evaluation of antiobiotic LL—D42067α as an anticoccidial agent in chicks

| | | | | Percent Chicks with Reduced Lesions | |
|---|---|---|---|---|---|
| Compound | Concentration in Diet, ppm | No. Chicks Started | Percent Survival | E. tenella | E. acervulina |
| LL—D42046α | 5.0 | 5 | 60 | 100 | 100 |
| LL—D42067α | 2.5 | 5 | 100 | 100 | 100 |
| LL—D42067α | 1.25 | 5 | 100 | 80 | 80 |
| LL—D42067α | 0.75 | 5 | 100 | 40 | 60 |
| LL—D42067α | 0.4 | 5 | 100 | 0 | 0 |
| LL—D42067α | 0.2 | 5 | 80 | 0 | 0 |
| Infected Untreated Control | 0.0 | 20 | 25 | 0 | 0 |
| LL—D42067α | 1.0 | 4 | 100 | 100 | 100 |
| LL—D42067α | 0.75 | 5 | 100 | 100 | 100 |
| LL—D42067α | 0.50 | 5 | 100 | 100 | 100 |
| LL—D42067α | 0.25 | 5 | 80 | 20 | 60 |
| Infected Untreated Control | 0.0 | 20 | 40 | 0 | 0 |

TABLE X

Evaluation of antiobiotic LL—D42067β as an anticoccidial agent in chicks

| Compound | Concentration in Diet, ppm | No. Chicks Started | Percent Survival | Percent Chicks with Reduced Lesions E. tenella |
|---|---|---|---|---|
| LL—D42046β | 5.0 | 5 | 80 | 100 |
| LL—D42067β | 2.5 | 5 | 100 | 100 |
| LL—D42067β | 1.00 | 5 | 80 | 80 |
| LL—D42067β | 0.75 | 5 | 60 | 40 |
| LL—D42046β | 0.5 | 5 | 40 | 0 |
| Infected Untreated Control | 0.0 | 20 | 35 | 0 |

Example 8

Evaluation of test compounds as anticoccidial agents in chicks

The usefulness of antibiotic LL—D42067α is demonstrated by its anticoccidial activity against a variety of coccidia species causing disease in chicks according to the following test.

Eighty times the recommended concentration of a commercial anticoccidial vaccine containing mixed species of *Eimeria* was presented by direct inoculation into the crops of all chicks. Chicks were ten days of age at time of inoculation. Two days before inoculation, medicated with feed with several levels of drug was presented to the various groups of chicks. Six days after inoculation, the test was terminated, and the intestinal tracks of the chicks were examined for lesions. A score was assigned to each chick depending upon the presence or absence of lesions and their severity, ranging from 0 to 4 per bird, times the number of birds per group. The percent reduction of lesions was calculated as follows:

$$100 - \frac{(\text{treated score (pooled)}}{(\text{control score (pooled)}} \times 100 \begin{matrix} ) \\ ) \end{matrix}$$

The results appear in Table XI below. These results show that lesions from five species of *Eimeria* in chicks can be prevented or significantly reduced at concentrations of drug as low as 0.2 ppm in the diet.

TABLE XI

Evaluation of antibiotic LL—D42067α for the control of coccidiosis in chicks

| Compound | Concentration in Diet, ppm | No. Chicks Started | Percent Reduction of Lesions | | | | |
|---|---|---|---|---|---|---|---|
| | | | E. tenella | E. acervulia | E. brunetti | E. necatrix | E. maxima |
| LL—D42067α | 1.25 | 15 | 100 | 95 | 100 | 100 | 100 |
| LL—D42067α | 1.0 | 15 | 100 | 91 | 100 | 100 | 100 |
| LL—D42067α | 0.75 | 15 | 74 | 86 | 100 | 100 | 100 |
| LL—D42067α | 0.40 | 15 | 52 | 62 | 89 | 100 | 72 |
| LL—D42067α | 0.20 | 15 | 31 | 24 | 65 | 82 | 0 |
| Infected Untreated Control | 0.0 | 15 | 7.7* | 14* | 9.3* | 1.7* | 11.7* |

* — Average lesion score per group of control chicks.

**Claims**

1. An animal feed or animal feed premix composition for the control of coccidiosis infections in meat-producing animals, said animal feed or animal feed premix comprising: a solid edible carrier; and 0.00001% by weight to 5.0% by weight of a compound of the formula,

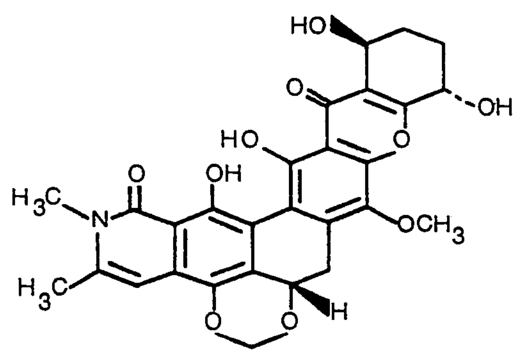

LL—D42067α

or

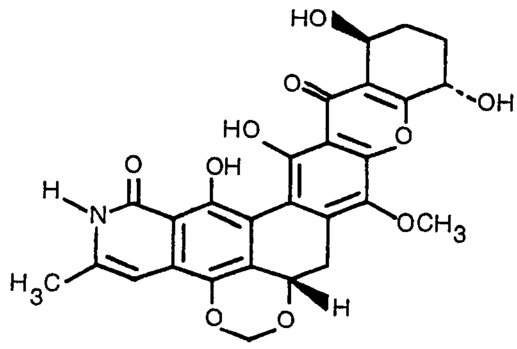

LL—D42067β

or pharmaceutically or pharmacologically acceptable salts thereof.

2. An animal feed or animal feed premix composition according to Claim 1, wherein said animals are poultry; and said composition comprises: 0.00001% by weight to 0.0005% by weight of a compound selected from

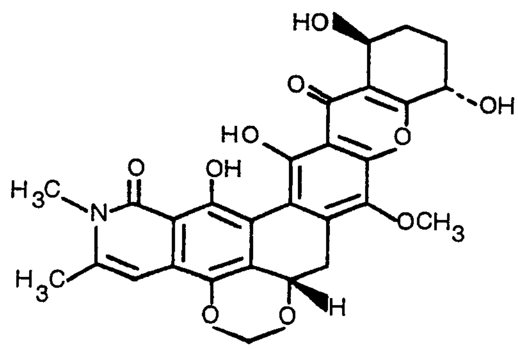

LL—D42067α

and

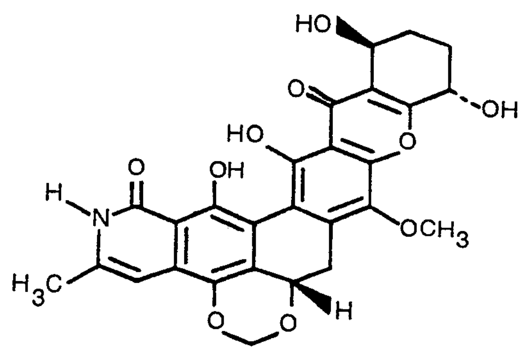

LL—D42067β

or pharmaceutically or pharmacologically acceptable salts thereof.

3. The compound designated LL—D42067α having:

a) the relative stereochemistry and structure:

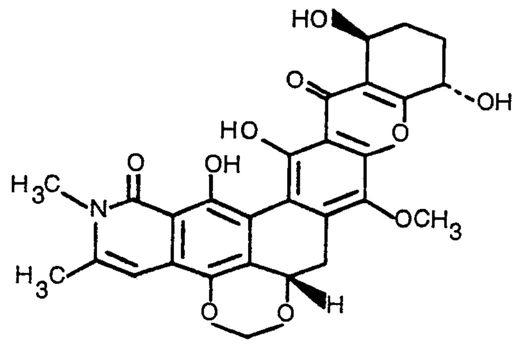

LL—D42067α

b) a molecular weight of 535 (FAB—MS);
c) a molecular formula: $C_{28}H_{25}NO_{10}$;
d) a specific optical rotation: $[\alpha]_D^{26}$ = +836±40°
e) characteristic ultraviolet absorption spectra as shown in Figure I of the attached drawings;
f) a characteristic infrared absorption spectrum as shown in Figure II of the attached drawings;
g) a characteristic proton nuclear magnetic resonance spectrum as shown in Figure III of the attached drawings;
h) a characteristic carbon-13 nuclear magnetic resonance spectrum as shown in Figure IV of the attached drawings with significant peaks at:
20.4; 25.4; 25.8; 29.0; 30.4; 58.5; 61.6; 63.3; 71.7; 90.4; 100.0; 109.2; 109.7; 111.0; 113.7; 119.0; 125.8; 126.6; 134.9; 135.3; 136.1; 141.3; 147.9; 151.1; 152.5; 165.4; 165.6; 182.3; and
i) a characteristic proton to carbon-13 chemical shift correlation as shown in Figure V of the attached drawings.

4. The compound of designated LL—D42067β having:
a) the relative stereochemistry and structure:

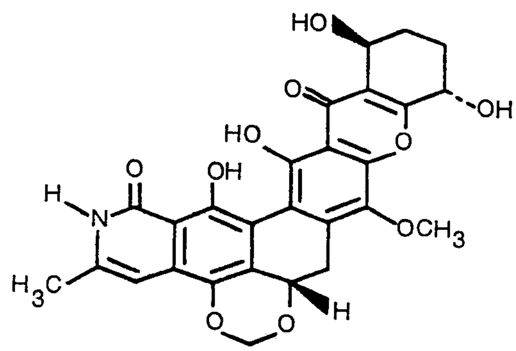

LL—D42067β

b) a molecular weight of 521 (FAB—MS);
c) a molecular formula: $C_{27}H_{23}NO_{10}$;
d) a specific optical rotation: $[\alpha]_D^{26}$ = +770±10° (C 0.165, DMF);
e) characteristic ultraviolet absorption spectra as shown in Figure VI of the attached drawings;
f) a characteristic infrared absorption spectrum as shown in Figure VIII of the attached drawings;
g) a characteristic proton nuclear magnetic resonance spectrum as shown in Figure VIII of the attached drawings; and
h) a characteristic carbon-13 nuclear magnetic resonance spectrum as shown in Figure IX of the attached drawings with significant peaks at:
19.1; 25.9; 26.9; 29.7; 62.0; 62.2; 65.0; 72.8; 91.1; 100.1; 110.5; 110.6; 111.7; 114.7; 119.2; 127.2; 128.8; 136.1; 136.3; 137.0; 138.1; 148.9; 151.9; 153.0; 165.3; 167.2; 183.6.

5. A process for producing antibiotic LL—D42067α and LL—D42067β which comprises aerobically fermenting the organism *Actinomadura madurae* subspecies *simaoensis,* having the identifying characteristics of NRRL 15734 or an antiobiotic LL—D42067α or LL—D42067β-producing mutant thereof, in a sterile liquid medium containing assimilable sources of carbon, nitrogen and inorganic anion and cation salts, until a substantial amount of LL—D42067α or LL—D42067β is produced in said medium and then recovering the antiobiotic therefrom.

6. A process for producing antibiotic LL—D42067α and LL—D42067β which comprises aerobically fermenting a sterile liquid medium containing assimilable sources of carbon, nitrogen and inorganic anion and cation salts, which medium has been inoculated with a viable culture of the organism *Actinomadura madurae* subspecies *simaoensis,* having the identifying characteristics of NRRL 15734 or an antibiotic LL—D42067α or LL—D42067β-producing mutant thereof, maintaining said fermentation culture with sterile aeration and agitation at a temperature of 24—32°C, at a pH of 7.0—7.6, for a period of 90—200 hours, harvesting the mash and extracting and antibiotic.

7. The biologically pure culture of the microorganism *Actinomadura madurae* subspecies *simaoensis,* having the identifying characteristics of NRRL 15734, said culture being capable of producing antibiotic LL—D42067α and LL—D42067β in recoverable quantities upon fermentation in an aqueous nutrient medium containing assimilable sources of carbon, nitrogen and inorganic anion and cation salts.

8. The biologically pure culture of the microorganism *Actinomadura madurae* subspecies *simaoensis* according to Claim 7, wherein said microorganism has been subjected to mutagenic means such that the microorganism is genetically altered but still retains the ability to synthesize antibiotics LL—D42067α or LL—D42067β.

**Patentansprüche**

1. Tierfutterzusammensetzung oder Tierfutterpremixzusammensetzung zur Bekämpfung von Kokkzidiosisinfektionen bei fleischerzeugenden Tieren, wobei das Tierfutter oder das Tierfutterpremix umfaßt:

einen festen, eßbaren Träger und 0,00001 Gew.-% bis 5,0 Gew.-% einer Verbindung der Formel,

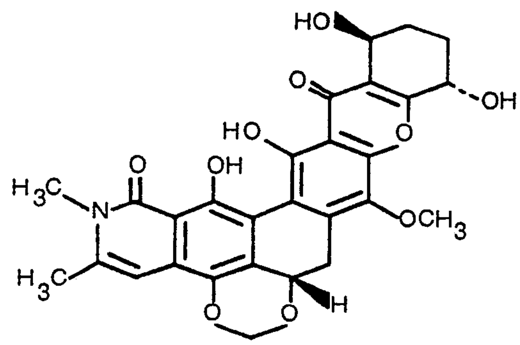

LL–D42067α

oder

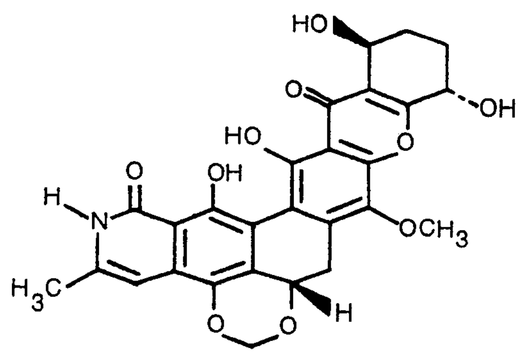

LL–D42067β

oder pharmazeutisch oder pharmakologisch akzeptable Salze derselben.

2. Tierfutter- oder Tierfutterpremixzusammensetzung gemäß Anspruch 1, wobei es sich bei den Tieren um Geflügel handelt und die Zusammensetzung umfaßt:

0,00001 Gew.-% bis 0,0005 Gew.-% einer Verbindung ausgewählt aus

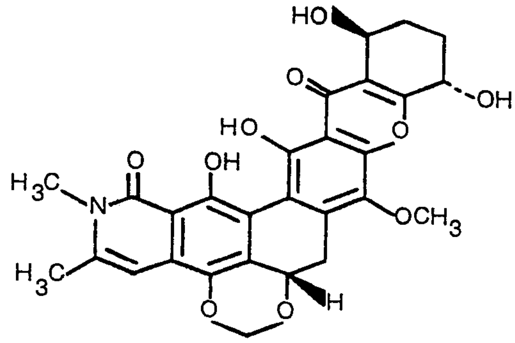

LL–D42067$\alpha$

und

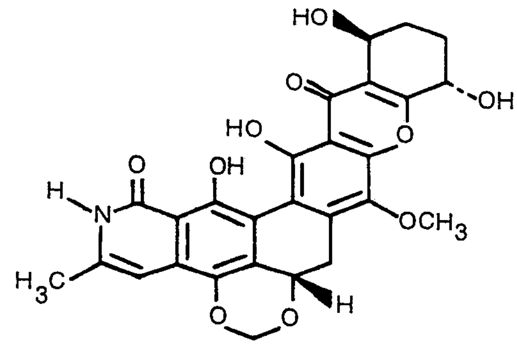

LL–D42067$\beta$

oder pharmazeutisch oder pharmakologisch akzeptable Salze derselben.

3. Die LL—D42067α beizeichnete Verbindung mit folgenden Eigenschaften:

a) der relativen Stereochemie und Struktur:

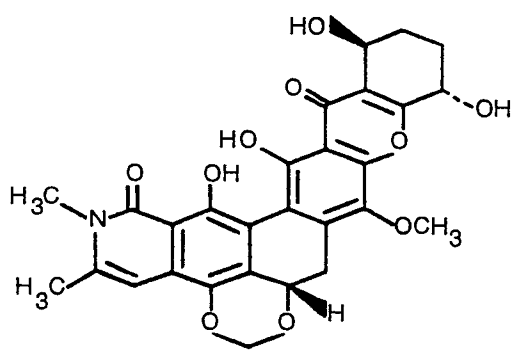

LL–D42067$\alpha$

b) ein Molekulargewicht von 535 (FAB—MS);

c) einer Molekülformel: $C_{28}H_{25}NO_{10}$;

d) einer spezifischen optischen Drehung: $[\alpha]_D^{26} = +836 \pm 40°$

e) charakteristische Ultraviolett-Absorbtionssprekten, wie sie in Figur I der beigefügten Zeichnungen gezeigt sind;

f) ein charakteristisches Infrarot-Absorptionsspektrum, wie es in Figur II der beigfügten Zeichnungen gezeigt ist;

g) ein charakteristisches Protonen-kernmagnetisches Resonanzspektrum, wie es in Figur III der beifügten Zeichnungen zegeigt ist;

h) ein charakteristisches Kohlenstoff-13-kernmagnetisches Resonanzspektrum, wie es in Figur IV der

beigefügten Zeichnungen gezeigt ist mit signifikanten Signalen bei: 20.4; 25.4; 25.8; 29.0; 30.4; 58.5; 61.6; 63.3; 71.7; 90.4; 100.0; 109.2; 109.7; 111.0; 113.7; 119.0; 125.8; 126.6; 134.9; 135.3; 136.1; 141.3; 147.9; 151.1; 152.5; 165.4; 165.6; 182.3; und

i) einer charakteristischen Protonen-zu-Kohlenstoff-13-chemischer Verschiebungskorelation, wie sie in Figur V der beigefügten Zeichnungen gezeigt ist.

4. Die LL—D42067β beizeichnete Verbindung mit folgenden Eigenschaften:

a) der relativen Stereochemie und Struktur:

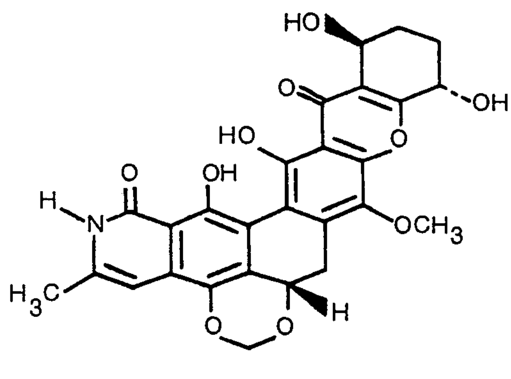

LL—D42067β

b) einem Molekulargewicht von 521 (FAB—MS);

c) einer Molekülformel: $C_{27}H_{23}NO_{10}$;

d) einer spezifischen optischen Drehung: $[\alpha]_D^{26} = +770 \pm 10°$ (C 0.165, DMF);

e) charakteristische Ultraviolett-Absorbtionssprekten, wie sie in Figur IV der beigefügten Zeichnungen gezeigt sind;

f) ein charakteristisches Infrarot-Absorptionsspektrum, wie es in Figur VII der beigefügten Zeichnungen gezeigt ist;

g) ein charakteristisches Protonen-kernmagnetisches Resonanzspektrum, wie es in Figur VIII der beigfügten Zeichnungen gezeigt ist; und

h) ein charakteristisches Kohlenstoff-13-kernmagnetisches Resonanzspektrum, wie es in Figur IX der beigefügten Zeichnungen gezeigt ist mit signifikanten Signalen bei: 19.1; 25.9; 26.9; 29.7; 62.0; 62.2; 65.0; 72.8; 91.1; 100.1; 110.5; 110.6; 111.7; 114.7; 119.2; 127.2; 128.8; 136.1; 136.3; 137.0; 138.1; 148.9; 151.9; 153.0; 165.3; 167.2; 183.6.

5. Verfahren zur Herstellung von antibiotischem LL—D42067α und LL—D42067β, umfassend die aerobische Fermentierung des Organismus *Actinomadura madurae* Subspezies *simaoensis* mit den identifizierenden Charakteristika von NRRL 15734 oder einer antibiotisches LL—D42067α oder LL—D42067β erzeugenden Mutanten desselben in einem sterilen flüssigen Medium, enthaltend assimilierbare Quellen von Kohlenstoff, Stickstoff und anorganischen Anionen- und Kationsalzen, bis eine wesentliche Menge LL—D42067α oder LL—D42067β in dem Medium erzeugt ist, und anschließend die Isolierung des Antibiotikum aus demselben.

6. Verfahren zur Herstellung von antibiotischem LL—D42067α und LL—D42067β, umfassend die aerobische Fermentierung eines sterilen flüssigen Mediums, enthaltend assimilierbare Quellen von Kohlenstoff, Stickstoff und anorganischen Anionen- und Kationensalzen, wobei das Medium inokkuliert wurde mit einer lebensfähigen Kultur des Organismus *Actinomadura madurae* Subspezies *simaoensis*, mit den identifizierenden Charakteristika von NRRL 15734 oder einer antibiotisches LL—D42067α oder LL—D42067β erzeugenden Mutante desselben, Halten der erwähnten Fermentationskultur mit steriler Belüftung und Rühren bei einer Temperatur 24—32°C bei einem pH von 7,0 bis 7,6 während eines Zeitraums von 90 bis 200 Stunden, Ernten der Maische und Extraktion des Antibiotikum.

7. Die biologische reine Kultur des Mikroorganismus *Actinomadura madurae* Subspezies *simaoensis*, mit den identifizierenden Charakteristika von NRRL 15734, wobei die Kultur in der Lage ist zur Erzeugung von antibiotischem LL—D42067α und LL—D42067β in isolierbaren Mengen bei Fermentation in einem wässrigen Nährmedium enthaltend assimilierbare Quellen von Kohlenstoff, Stickstoff und anorganischen Anionen- und Kationensalzen.

8. Die biologisch reine Kultur des Mikroorganismus *Actinomadura madurae* Subspezies *simaoensis*, gemäß Anspruch 7, wobei der erwähnte Mikroorganismus mutagenen Mitteln unterworfen wurde derart, daß der Mikroorganismus genetisch verändert wurde, jedoch seine Fähigkeit, antibiotisches LL—D42067α und LL—D42067β zu synthetisieren, behalten hat.

**Revendications**

1. Composition d'aliment pour animaux ou de prémélange alimentaire pour animaux pour la lutte contre les infections à coccidies chez les animaux producteurs de viande, ledit aliment pour animaux ou

ledit prémélange alimentaire pour animaux comprenant: un support comestible solide; et 0,00001% en poids à 5,0% en poids d'un composé de formule,

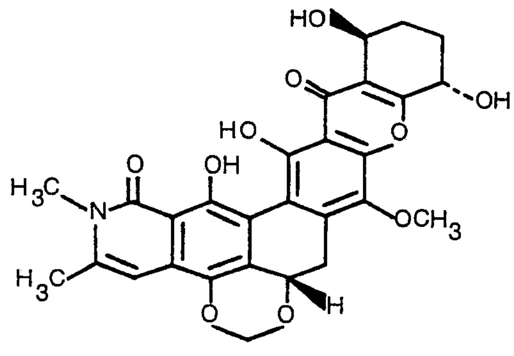

LL–D42067α

ou

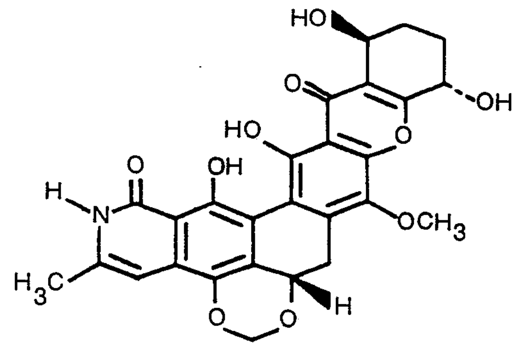

LL–D42067β

ou leurs sels pharmaceutiquement ou pharmacologiquement acceptables.

2. Composition d'aliment pour animaux ou de prémélange alimentaire pour animaux selon la revendication 1, où lesdits animaux sont des volailles et ladite composition comprend: 0,00001% en poids à 0,0005% en poids d'un composé choisi parmi

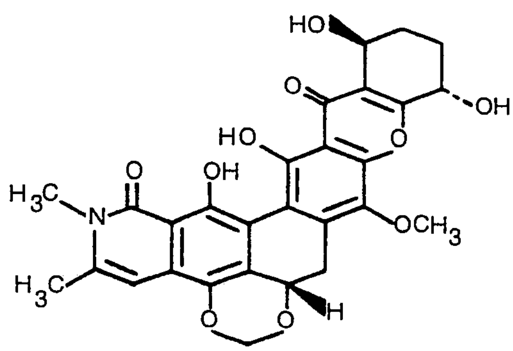

LL–D42067α

et

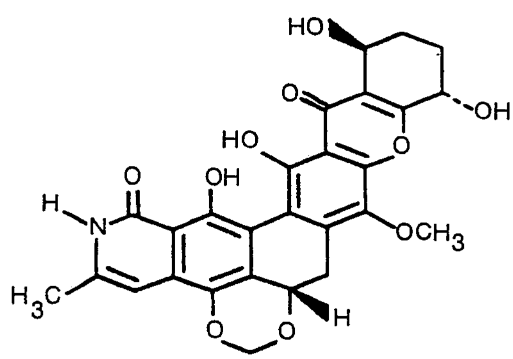

LL–D42067β

ou leurs sels pharmaceutiquement ou pharmacologiquement acceptables.

3. Composé appelé LL—D42067α ayant:

a) la stéréochimie et la structure relatives:

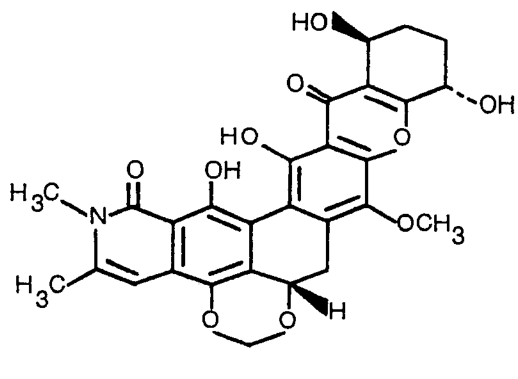

LL—D42067α

b) un poids moléculaire de 535 (bombardement par atomes rapidesspectre de masse);

c) une formule moléculaire: $C_{28}H_{25}NO_{10}$;

d) une rotation optique spécifique: $[\alpha]_D^{26} = +836\pm40°$;

e) les spectres caractéristiques d'absorbtion ultraviolette illustrés par la figure I des dessins annexés;

f) un spectre caractéristique d'absorption infrarouge comme illustré par la figure II des dessins annexés;

g) un spectre caractéristique de résonance magnétique nucléaire protonique comme illustré par la figure III des dessins annexés;

h) un spectre caractéristique de résonance magnétique nucléaire du carbone-13 comme illustré par la figure IV des dessins annexés avec des pics significatifs à: 20,4; 25,4; 25,8; 29,0; 30,4; 58,5; 61,6; 63,3; 71,7; 90,4; 100,0; 109,2; 109,7; 111,0; 113,7; 119,0; 125,8; 126,6; 134,9; 135,3; 136,1; 141,3; 147,9; 151,1; 152,5; 165,4; 165,6; et 182,3; et

i) une corrélation caractéristique entre le déplacement chimique protonique et celui du carbone 13 comme illustré par la figure V des dessins annexés.

4. Composé appelé LL—D42067β ayant:

a) la stéréochimie et la structure relatives:

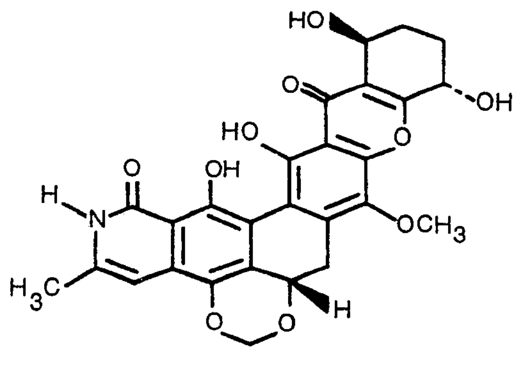

LL—D42067β

b) un poids moléculaire de 521 (bombardment par atomes rapidesspectre de masse);

c) une formule moléculaire: $C_{27}H_{23}NO_{10}$;

d) une rotation optique spécifique: $[\alpha]_D^{26} = +770\pm10°$ (C = 0,165; DMF);

e) les spectres caractéristiques d'absorbtion ultraviolette comme illustré par la figure VI des dessins annexés;

f) un spectre caractéristique d'absorption infrarouge comme illustré par la figure VIII des dessins annexés;

g) un spectre caractéristique de résonance magnétique nucléaire protonique comme illustré par la figure VIII des dessins annexés; et

h) un spectre caractéristique de résonance magnétique nucléaire du carbone 13 comme illustré par la figure IX des dessins annexés avec des pics significatifs à: 19,1; 25,9; 26,9; 29,7; 62,0; 62,2; 65,0; 72,8; 91,1; 100,1; 110,5; 110,6; 111,7; 114,7; 119,2; 127,2; 128,8; 136,1; 136,3; 137,0; 138,1; 148,9; 151,9; 153,0; 165,3; 167,2; et 183,6.

5. Procédé pour la production des antibiotiques LL—D42067α et LL—D42067β, qui comprend la fermentation aérobie de l'organisme *Actinomadura madurae sous-espèce simaoensis,* ayant les caractéristiques d'identification de NRRL 15734, ou un mutant de celui-ci produisant l'antibiotique LL—D42067α ou LL—D42067β, dans un milieu liquide stérile contenant des sources assimilables de carbone, d'azote et des sels minéraux anioniques et cationiques, jusqu'à ce qu'une quantité notable de LL—D42067α ou LL—D42067β soit produite dans lesdit milieu, puis la récupération de l'antibiotique à partir de celui-ci.

6. Procédé pour la production des antibiotiques LL—D42067α et LL—D42067β, qui comprend la fermentation aérobie d'un milieu liquide stérile contenant des sources assimilables de carbone, d'azote et de sels minéraux anioniques et cationiques, lequel milieu a été ensemencé avec une culture viable de l'organisme *Actinomadura madurae sous-espèce simaoensis,* ayant les caractéristiques d'identification de NRRL 15734, ou un mutant producteur de l'antibiotique LL—D42067α ou LL—D42067β de celui-ci, en maintenant ladite culture par fermentation avec aération stérile et agitation à une température de 24 à 32°C et à un pH de 7,0 à 7,6, pendant une période de 90 à 200 heures, la récolte du produit fermentaire et l'extraction de l'antibiotique.

7. Culture biologiquement pure du microoranisme *Actinomadura madurae* sous-espèce *simaoensis,* ayant les caractéristiques d'identification de NRRL 15734, ladite culture pouvant produire les antibiotiques LL—D42067α et LL—D42067β en des quantités récupérables par fermentation dans un milieu nutritif aqueux contenant des sources assimilables de carbone, d'azote et de sels minéraux anioniques et cationiques.

8. Culture biologiquement pure du microorganisme *Actinomadura madurae* sous-espèce *simaoensis* selon la revendication 7, dans laquelle ledit microorganisme a été soumis à un moyen mutagène, si bien que le microorganisme présente une altération génétique mais conserve la capacité de synthétiser l'antibiotique LL—D42067α ou LL—D42067β.

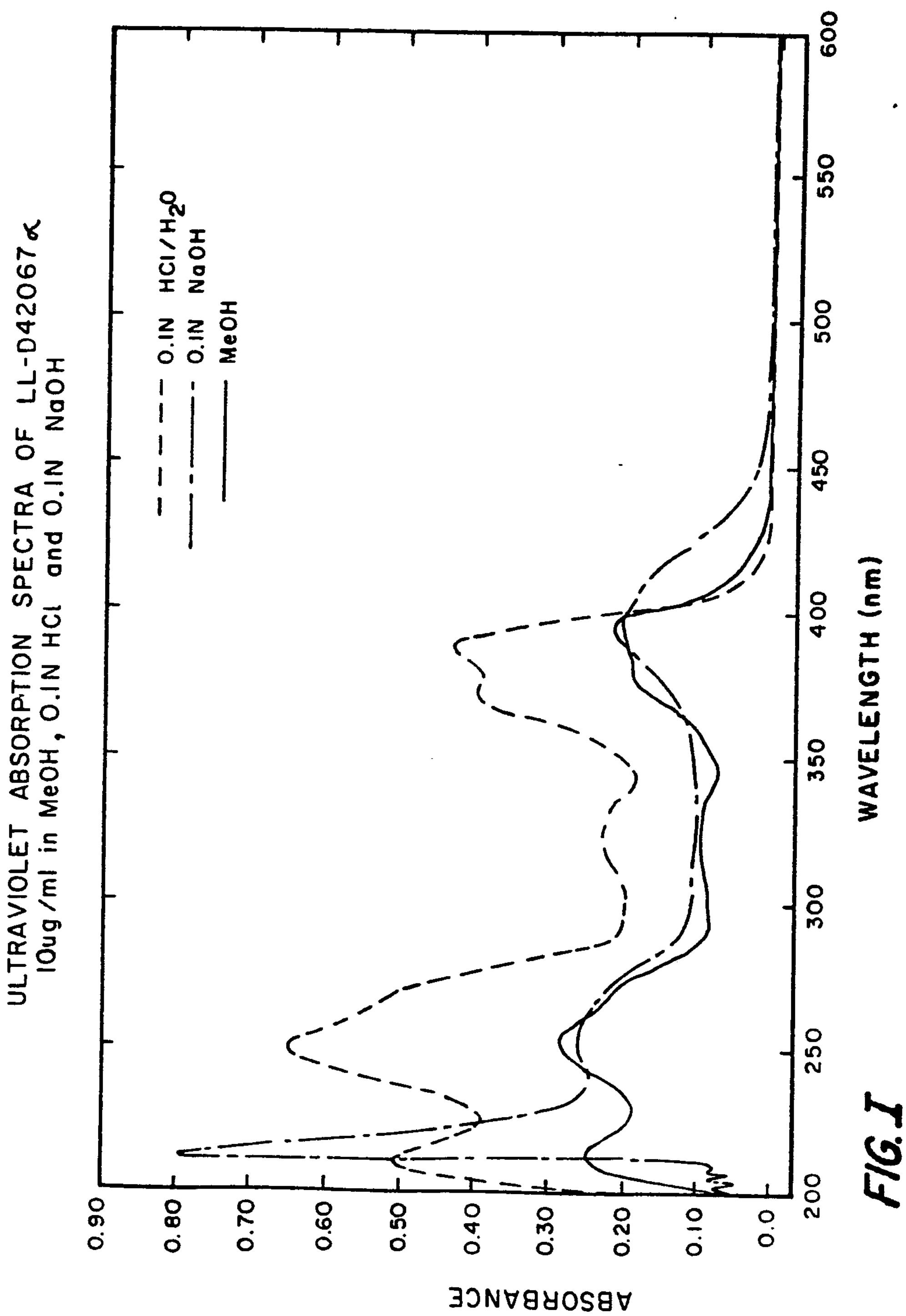
ULTRAVIOLET ABSORPTION SPECTRA OF LL-D42067α
10ug/ml in MeOH, 0.1N HCl and 0.1N NaOH
0.1N HCl/$H_2O$
0.1N NaOH
MeOH
ABSORBANCE
0.0
0.10
0.20
0.30
0.40
0.50
0.60
0.70
0.80
0.90
200
250
300
350
400
450
500
550
600
WAVELENGTH (nm)

FIG. I

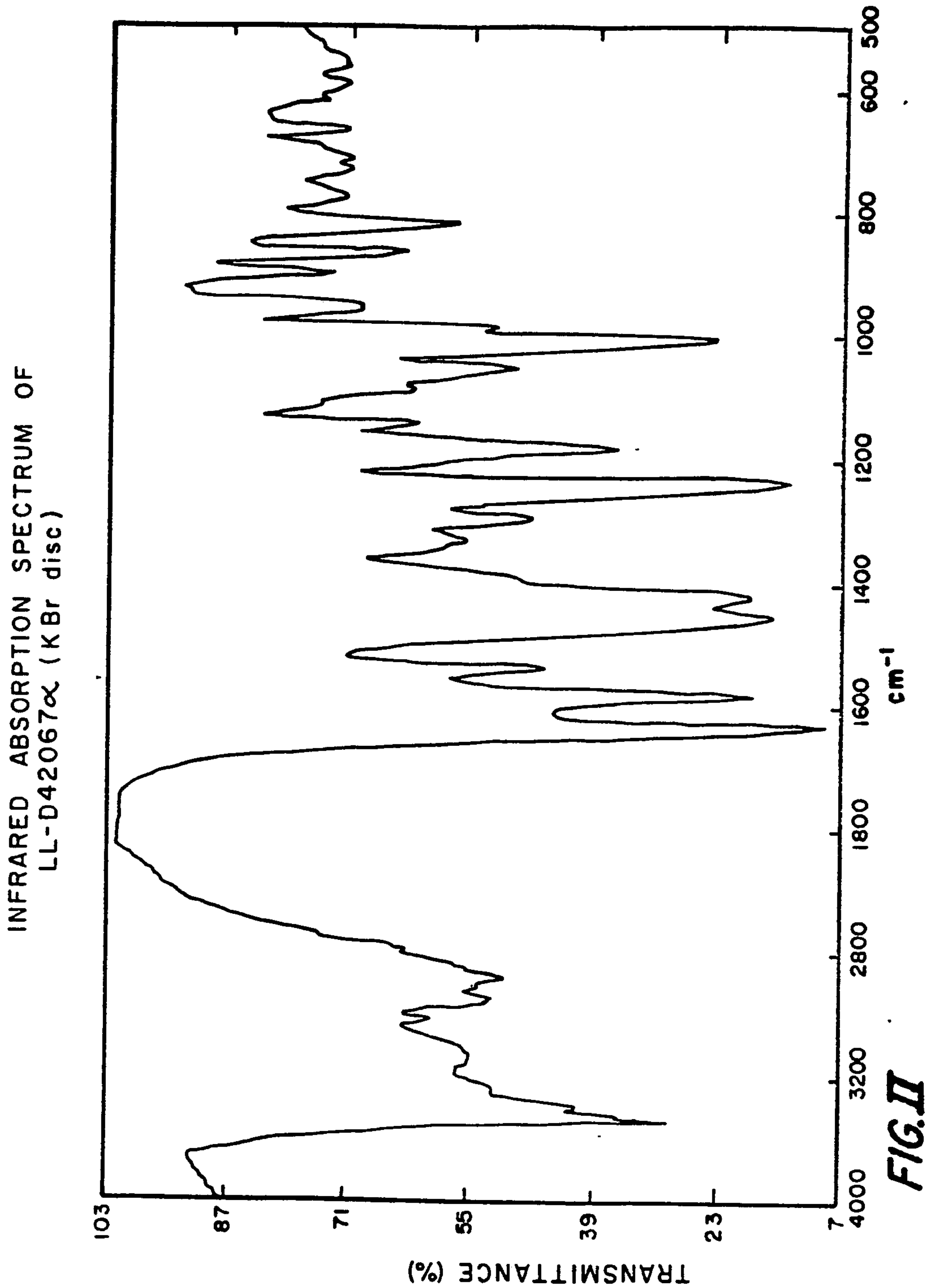
INFRARED ABSORPTION SPECTRUM OF
LL-D42067α (KBr disc)
TRANSMITTANCE (%)
103
87
71
55
39
23
7
4000
3200
2800
1800
1600
1400
1200
1000
800
600
500
cm-1

*FIG.II*

PROTON NUCLEAR MAGNETIC RESONANCE SPECTRUM
OF LL-D42067α IN $CDCl_3$ SOLUTION

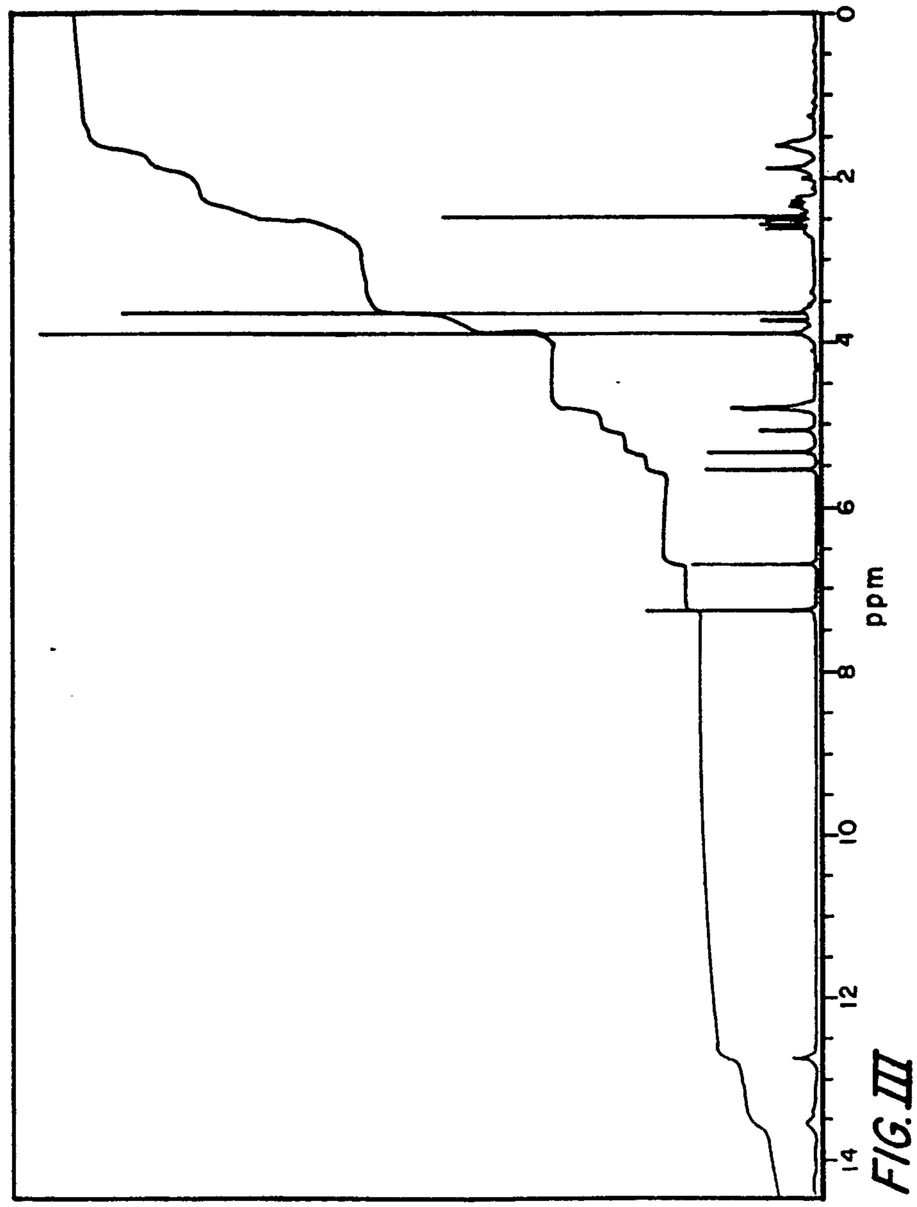


FIG. III

CARBON-13 NUCLEAR MAGNETIC RESONANCE SPECTRUM OF LL-D42067α IN DMSO SOLUTION

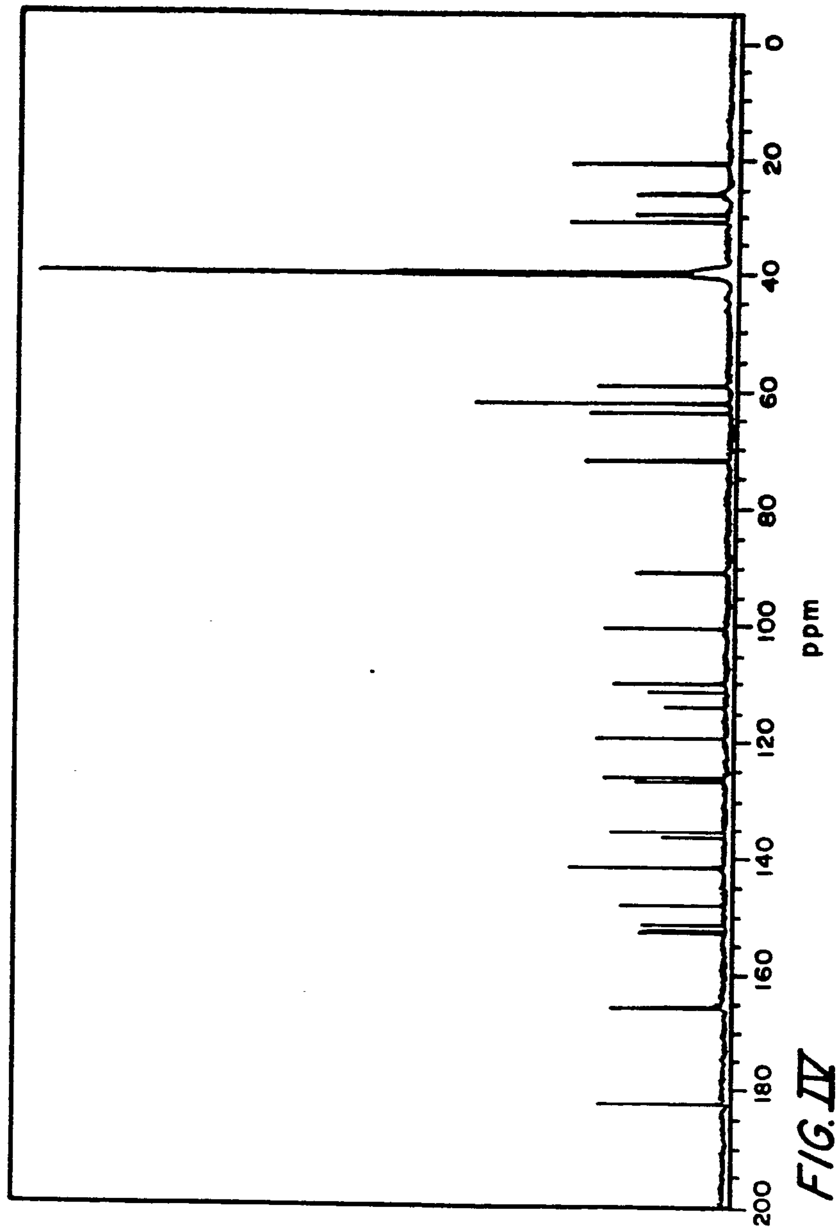


FIG. IV

PROTON TO CARBON-13 CHEMICAL SHIFT CORRELATION OF LL-D42067α IN DMSO SOLUTION

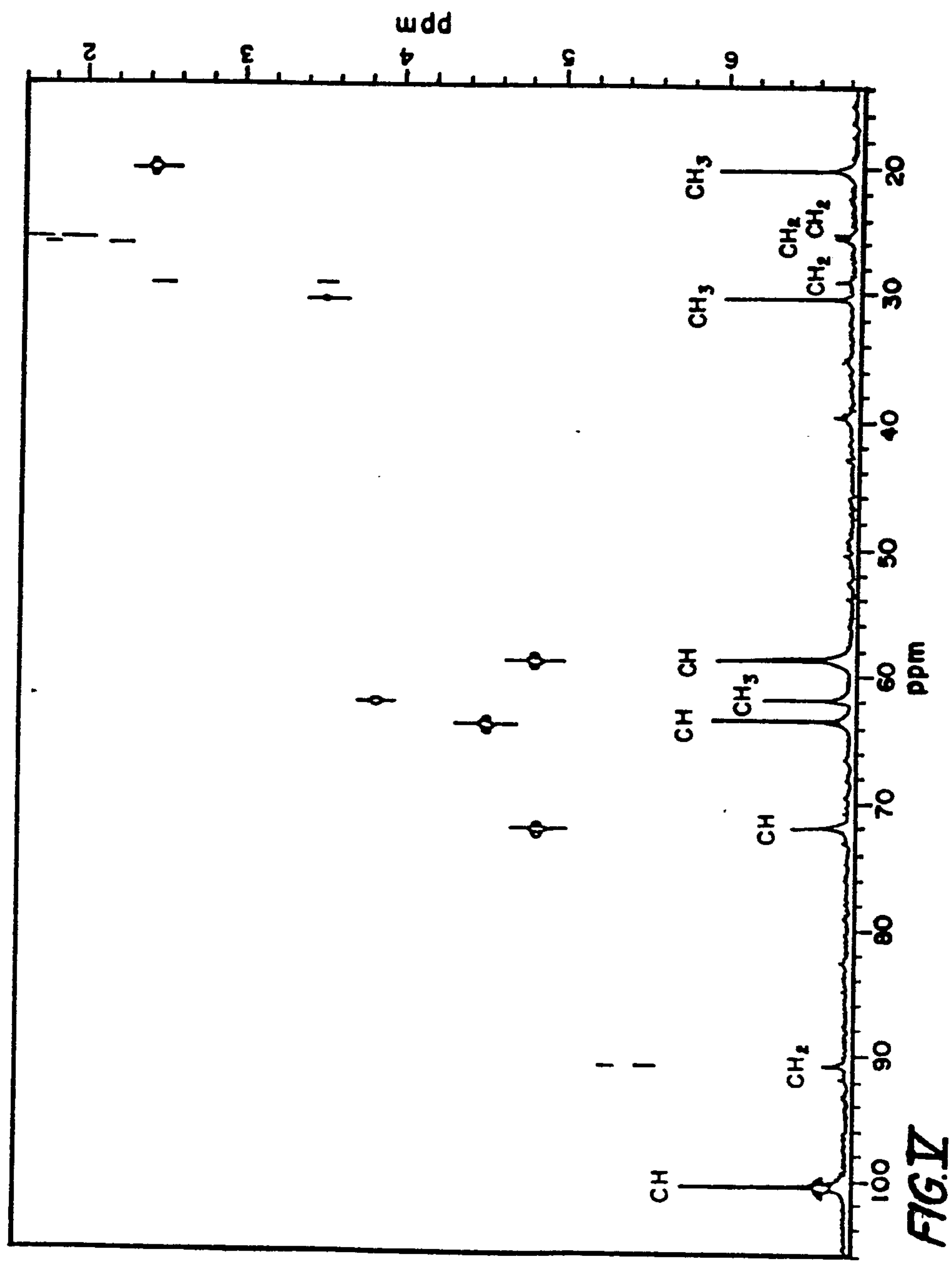


FIG. V

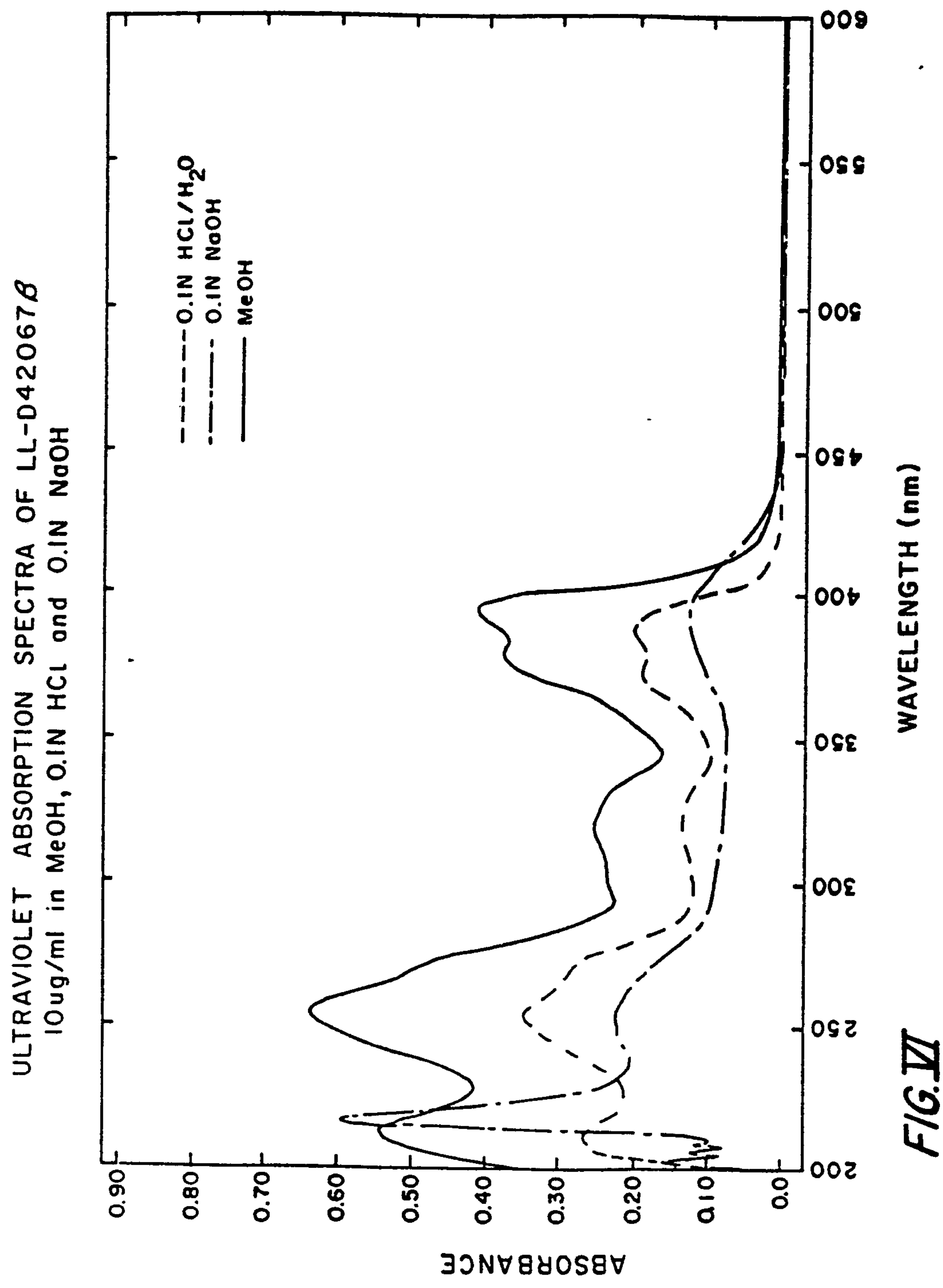
ULTRAVIOLET ABSORPTION SPECTRA OF LL-D42067β
10ug/ml in MeOH, O.IN HCL and O.IN NaOH
O.IN HCl/H2O
O.IN NaOH
MeOH
ABSORBANCE
0.90
0.80
0.70
0.60
0.50
0.40
0.30
0.20
0.10
0.0
200
250
300
350
400
450
500
550
600
WAVELENGTH (nm)

FIG. VII

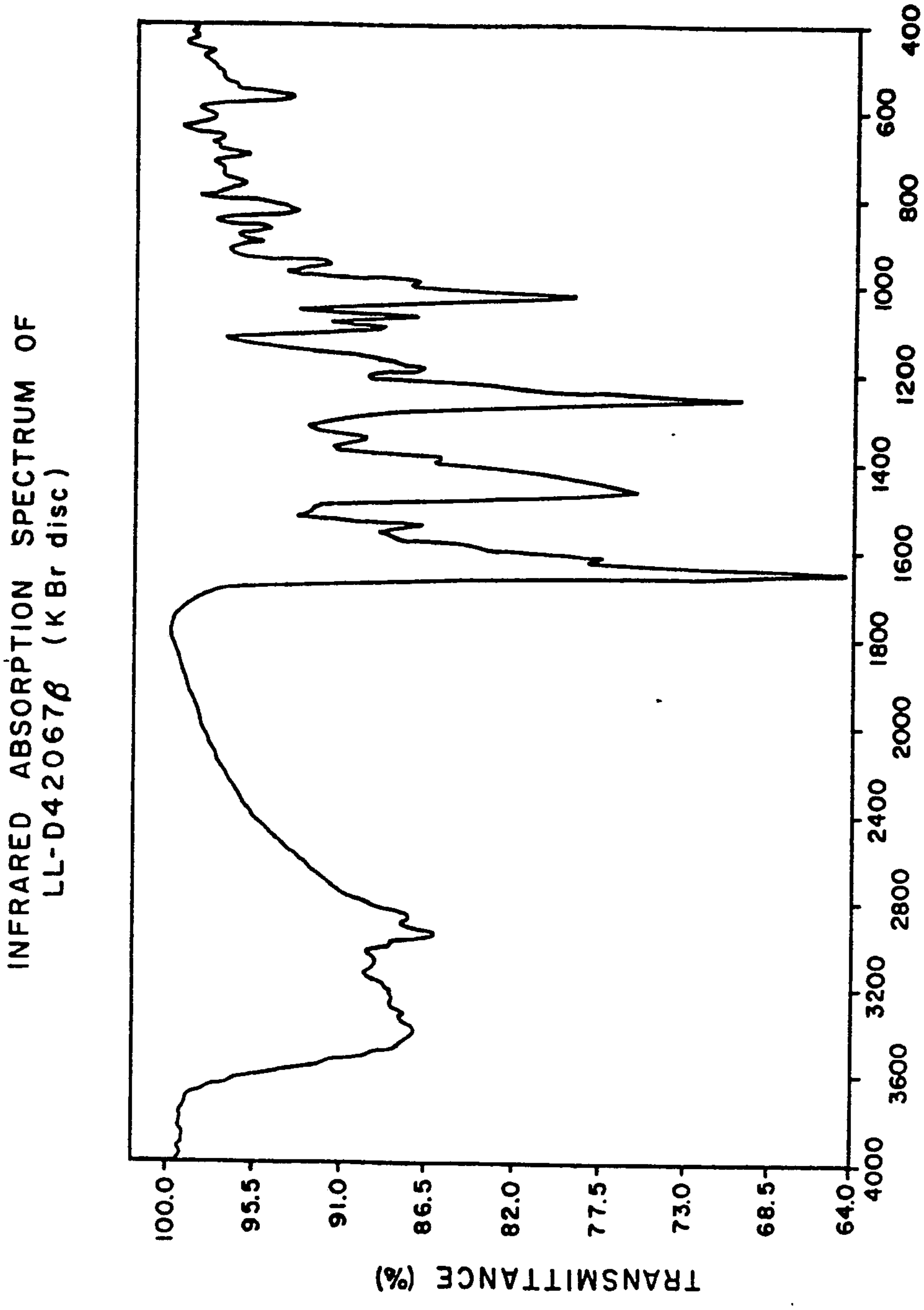
INFRARED ABSORPTION SPECTRUM OF
LL-D42067β (KBr disc)
TRANSMITTANCE (%)
100.0
95.5
91.0
86.5
82.0
77.5
73.0
68.5
64.0
4000
3600
3200
2800
2400
2000
1800
1600
1400
1200
1000
800
600
400
$cm^{-1}$

FIG. VIII

PROTON NUCLEAR MAGNETIC RESONANCE SPECTRUM
OF LL-D42067β IN $CDCl_3$ SOLUTION

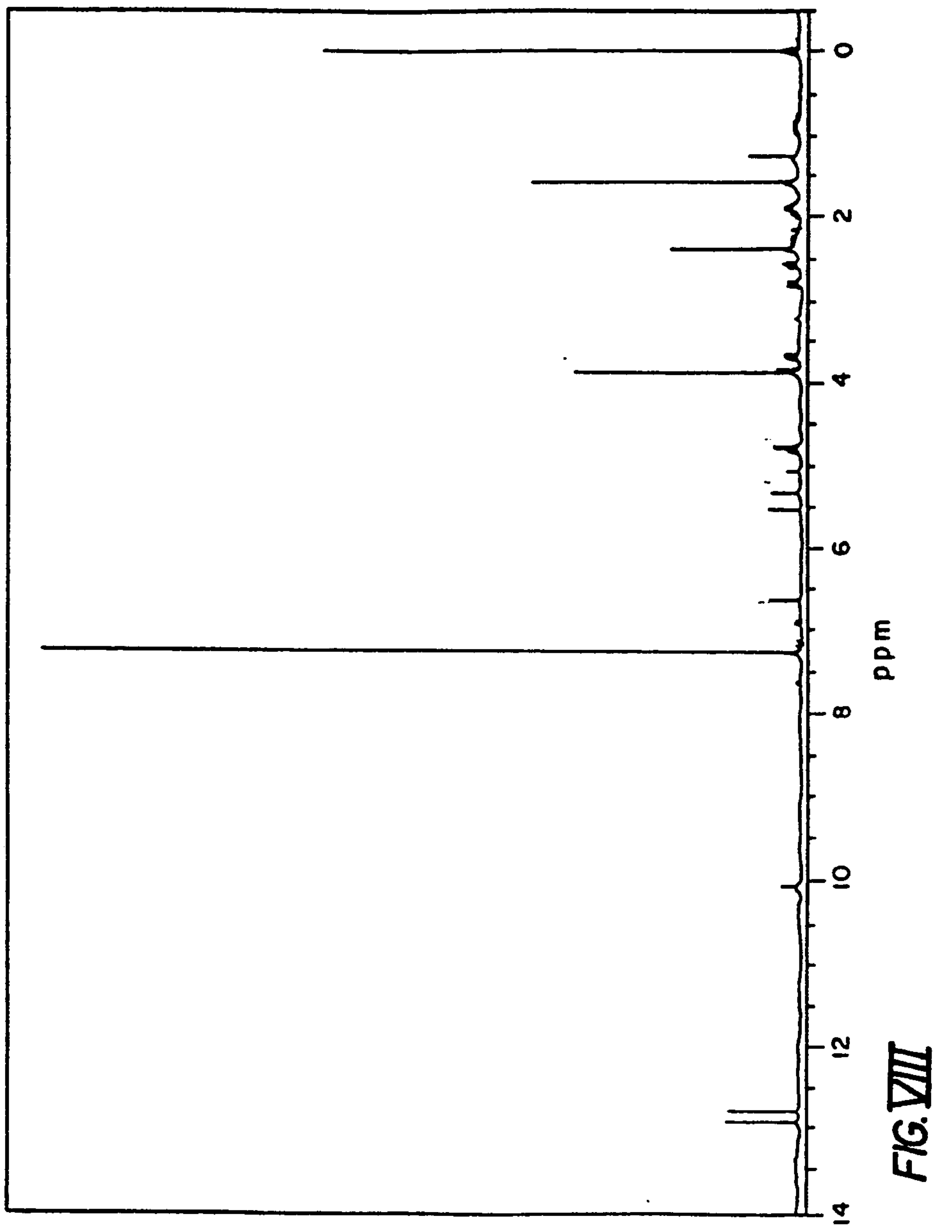


FIG. VIII

CARBON-13 NUCLEAR MAGNETIC RESONANCE SPECTRUM OF LL-D42067β IN $CD_3OD/CDCl_3$ SOLUTION

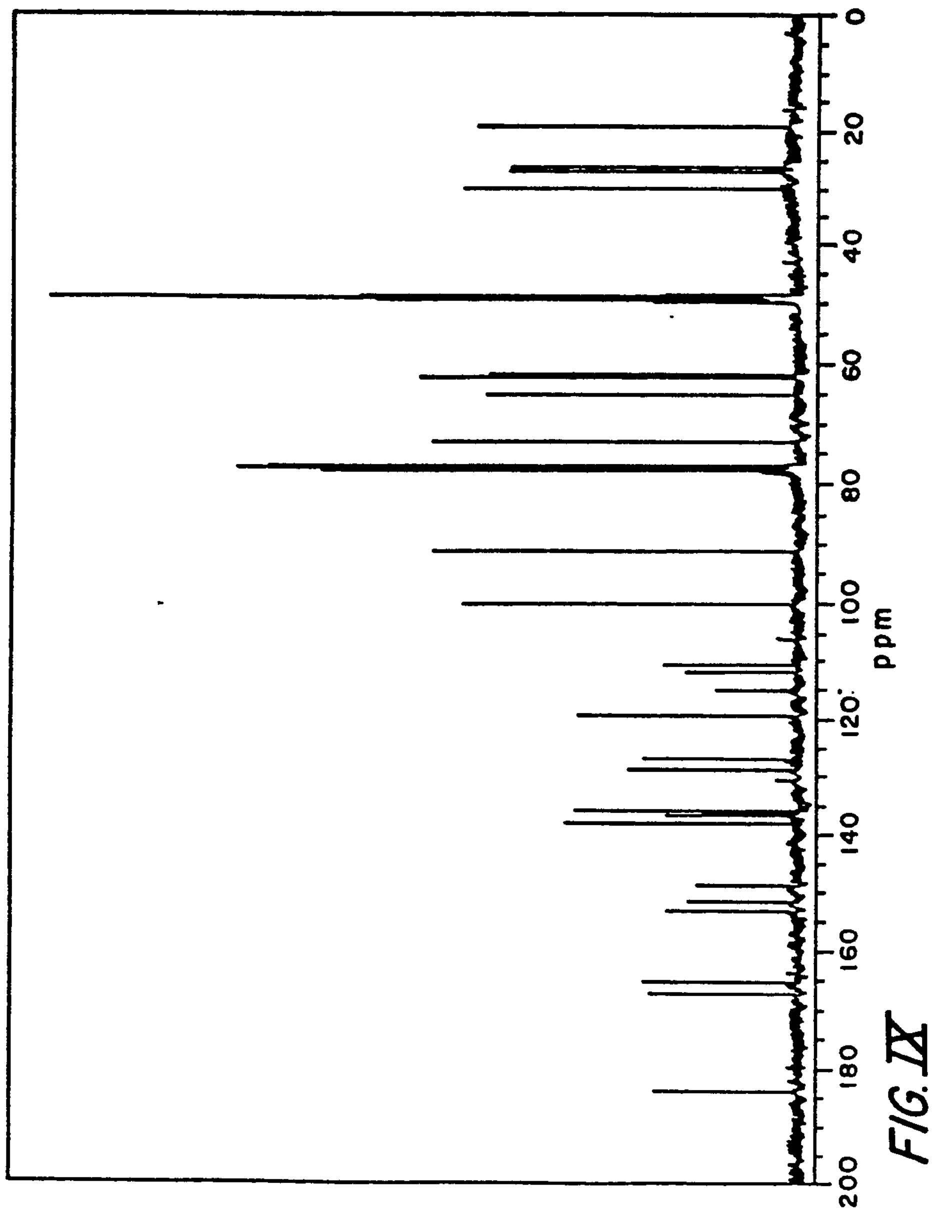


FIG. IX